(19) Europäisches Patentamt
European Patent Office
Office européen des brevets

(11) EP 4 417 599 A1

(12) **EUROPEAN PATENT APPLICATION**
published in accordance with Art. 153(4) EPC

(43) Date of publication:
**21.08.2024 Bulletin 2024/34**

(21) Application number: **22880400.1**

(22) Date of filing: **14.10.2022**

(51) International Patent Classification (IPC):
***C07D 209/26*** *(2006.01)* ***C07C 317/44*** *(2006.01)*
***A61K 31/405*** *(2006.01)* ***A61K 31/216*** *(2006.01)*
***A61P 35/00*** *(2006.01)* ***A61P 35/02*** *(2006.01)*
***A61P 29/00*** *(2006.01)*

(52) Cooperative Patent Classification (CPC):
**A61K 31/216; A61K 31/405; A61P 29/00; A61P 35/00; A61P 35/02; C07C 317/44; C07D 209/26**

(86) International application number:
**PCT/CN2022/125293**

(87) International publication number:
**WO 2023/061464 (20.04.2023 Gazette 2023/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.10.2021 CN 202111202003**

(71) Applicant: **Beijing Anjianxi Bio-Medical Technology Co., Ltd.**
**Beijing 100036 (CN)**

(72) Inventors:
- **XU, Yao**
**Beijing 100036 (CN)**
- **WANG, Haiyong**
**Beijing 100036 (CN)**
- **MI, Chunlai**
**Beijing 100036 (CN)**
- **MEI, Yunhong**
**Beijing 100036 (CN)**

(74) Representative: **Maiwald GmbH**
**Elisenhof**
**Elisenstraße 3**
**80335 München (DE)**

(54) **2,3-DIMETHOXY-5-METHYL-1,4-BENZOQUINONE ALKYL ALCOHOL DERIVATIVE AND USE THEREOF**

(57) Disclosed is a 2,3-dimethoxy-5-methyl-1,4-benzoquinone alkyl alcohol derivative, a pharmaceutical composition containing the compound, and the use of the compound in the prevention and treatment of tumors.

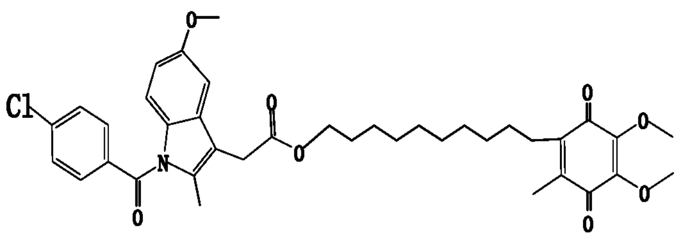

(I)

EP 4 417 599 A1

## Description

### TECHNICAL FIELD

The present disclosure relates to the pharmaceutical field, more particularly, relates to a 2,3-dimethoxy-5-methyl-1,4-benzoquinone alkyl alcohol derivative, pharmaceutical compositions comprising the compound as an active ingredient, and use of the compound in prevention and treatment of tumors.

### BACKGROUND

With the changes of human consumption structure and lifestyle, as well as the deterioration of living environment, the incidence rate of malignant tumors has increased rapidly and has become the most terrifying killer threatening human life and health. Anti-cancer research remains one of the most challenging and significant areas in life sciences.

Targeted drug therapy for cancer has made great progress, and has become the first choice particularly in the treatment of some lung cancers and non-solid tumor malignancies. However, cytotoxic drugs are still mainly used in clinical practice. In particular, the platinum-based drugs, including cisplatin, carboplatin, nedaplatin, oxaliplatin, and loplatin, are still the first-line treatment drugs for many common cancers. However, the platinum-based drugs have poor selectivity and strong side effects, among which, nephrotoxicity is one of the most common and influential side effects. The incidence rate of nephrotoxicity is 28-36% caused by cisplatin, 27% caused by carboplatin, and 10-15% caused by nedaplatin. The incidence rate and the extent of damage are proportional to the dosage, and the main clinical manifestations are increases in serum creatinine and urea nitrogen, etc.

Therefore, people have been looking forward to discovering new, safer and more effective anti-tumor drugs, particularly the anti-tumor drugs that can also increase the anti-tumor effect of platinum-based drugs and reduce the toxic and side effects of platinum-based drugs.

The present disclosure provides a 2,3-dimethoxy-5-methyl-1,4-benzoquinone alkyl alcohol derivative, which can simultaneously increase the anti-tumor effect of platinum-based drugs, reduce the toxic and side effects of platinum-based drugs, and has excellent therapeutic effect against a variety of tumors.

### SUMMARY

A first aspect of the present disclosure provides a 2,3-dimethoxy-5-methyl-1,4-benzoquinone alkyl alcohol derivative represented by formula (I) (referred to as "compound of formula (I)", "R01", or "RO1" briefly), or a solvate, a hydrate, a polymorph, a prodrug or an isotopic variant thereof, or a mixture thereof:

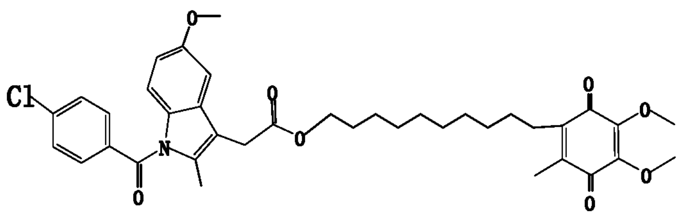

(I).

A second aspect of the present disclosure provides a pharmaceutical composition, comprising the compound of the first aspect, or a solvate, a hydrate, a polymorph, a prodrug or an isotopic variant thereof, and pharmaceutically acceptable excipient(s), and optionally other therapeutic agent(s).

A third aspect of the present disclosure relates to use of the compound of the first aspect, or a solvate, a hydrate, a polymorph, a prodrug or an isotopic variant thereof, or the pharmaceutical composition of the second aspect, in the manufacture of a medicament for the treatment and/or prevention of a cancer.

A fourth aspect of the present disclosure relates to a method of treating and/or preventing a cancer in a subject, comprising administering the compound of the first aspect, or a solvate, a hydrate, a polymorph, a prodrug or an isotopic variant thereof, or the pharmaceutical composition of the second aspect to the subject.

A fifth aspect of the present disclosure relates to the compound of the first aspect, or a solvate, a hydrate, a polymorph, a prodrug or an isotopic variant thereof, or the pharmaceutical composition of the second aspect, for use in the treatment and/or prevention of a cancer.

A sixth aspect of the present disclosure relates to the use of the third aspect, or the method of the fourth aspect, or the compound or the composition for use of the fifth aspect, wherein the cancer is selected from lung cancer, gastric cancer, esophageal cancer, and colorectal cancer.

A seventh aspect of the present disclosure relates to a combination, comprising the compound of formula (I), or a solvate, a hydrate, a polymorph, a prodrug or an isotopic variant thereof, and a platinum-based drug.

An eighth aspect of the present disclosure relates to a pharmaceutical composition, comprising the combination of the seventh aspect, and a pharmaceutically acceptable carrier, diluent or excipient.

A ninth aspect of the present disclosure relates to use of the combination of the seventh aspect in the manufacture of a medicament for the treatment and/or prevention of a cancer.

A tenth aspect of the present disclosure relates to a pharmaceutical product, comprising the compound of formula (I), or a solvate, a hydrate, a polymorph, a prodrug or an isotopic variant thereof, and a platinum-based drug, as a combination formulation for simultaneous, sequential or separate use in treatment.

An eleventh aspect of the present disclosure relates to a method of treating and/or preventing a cancer, comprising administering the compound of formula (I), or a solvate, a hydrate, a polymorph, a prodrug or an isotopic variant thereof, and a platinum-based drug simultaneously, sequentially, or separately to a patient.

A twelfth aspect of the present disclosure relates to use of the compound of formula (I), or a solvate, a hydrate, a polymorph, a prodrug or an isotopic variant thereof, in the manufacture of a medicament for the treatment and/or prevention of a cancer, wherein the treatment includes administering the compound of formula (I), or a solvate, a hydrate, a polymorph, a prodrug or an isotopic variant thereof, and a platinum-based drug simultaneously, sequentially, or separately to a patient.

A thirteenth aspect of the present disclosure relates to use of the compound of formula (I), or a solvate, a hydrate, a polymorph, a prodrug or an isotopic variant thereof, and a platinum-based drug in the manufacture of a medicament for the treatment and/or prevention of a cancer.

A fourteenth aspect of the present disclosure relates to use of the compound of formula (I), or a solvate, a hydrate, a polymorph, a prodrug or an isotopic variant thereof, in the manufacture of a medicament for the treatment and/or prevention of a cancer, wherein the medicament is used in a combination therapy with platinum-based drug(s).

A fifteenth aspect of the present disclosure relates to use of a platinum-based drug in the manufacture of a medicament for the treatment and/or prevention of a cancer, wherein the medicament is used in a combination therapy with the compound of formula (I), or a solvate, a hydrate, a polymorph, a prodrug or an isotopic variant thereof.

## BRIEF DESCRIPTION OF DRAWINGS

Figure 1 shows the effect of the compound of formula (I) on A549 cell proliferation detected by CCK-8, in which Figure 1A shows the effect of different concentrations of the compound of formula (I) co-cultured with A549 cells for 24 h on cell proliferation activity; Figure 1B shows the effect of different concentrations of the compound of formula (I) co-cultured with A549 cells for 48 h on cell proliferation activity; Figure 1C shows the effect of different concentrations of the compound of formula (I) co-cultured with A549 cells for 72 h on cell proliferation activity; Figure 1D shows the effect of different concentrations of the compound of formula (I) co-cultured for different lengths of time on A549 cell proliferation activity.

Figure 2 shows the effect of the compound of formula (I) on NCI-H460 cell proliferation detected by CCK-8, in which Figure 2A shows the effect of different concentrations of the compound of formula (I) co-cultured with NCI-H460 cells for 24 h on cell proliferation activity; Figure 2B shows the effect of different concentrations of the compound of formula (I) co-cultured with NCI-H460 cells for 48 h on cell proliferation activity; Figure 2C shows the effect of different concentrations of the compound of formula (I) co-cultured with NCI-H460 cells for 72 h on cell proliferation activity; Figure 2D shows the effect of different concentrations of the compound of formula (I) co-cultured for different lengths of time on NCI-H460 cell proliferation activity.

Figure 3 shows the effect of the compound of formula (I) on the proliferation of lung cancer cell lines detected by EDU proliferation assay. The compound of formula (I) at a concentration of 150 $\mu$M was co-cultured with the cells for 42 h, and then the FBS concentration in the FBS stimulation well was increased to 20%. The cells were cultured for another 6 h, and then detected using a Cell-Light EDUApollo567 kit. Figure 3A shows the effect of the compound of formula (I) on the proliferation of A549 cells; Figure 3B shows the effect of the compound of formula (I) on the proliferation of NCI-H460 cells; *: $P<0.05$; **: $P<0.01$.

Figure 4 shows the effect of different concentrations of the compound of formula (I) on the colony formation ability of A549 and NCI-H460 cells.

Figure 5 shows representative flow cytometry profiles of cell cycle distribution of A549 cells after being treated with different concentrations of the compound of formula (I) (0 $\mu$M, 10 $\mu$M, 30 $\mu$M, and 90 $\mu$M) for 24 h, 48 h and 72 h, indicating that the compound of formula (I) induces a decrease in the proportion of S phase in A549 cells.

Figure 6 shows representative flow cytometry profiles of cell cycle distribution of NCI-H460 cells after being treated with different concentrations of the compound of formula (I) (0 $\mu$M, 10 $\mu$M, 30 $\mu$M, and 90 $\mu$M) for 24 h, 48 h and

72 h, indicating that the compound of formula (I) induces a decrease in the proportion of S phase in NCI-H460 cells.

Figure 7 shows scatter plots of changes in apoptosis of A549 and NCI-H460 cells detected by flow cytometry after being treated with different concentrations of the compound of formula (I). Figures 7A, 7B, 7C, and 7D are representative cell apoptosis plots of A549 cells treated with different concentrations of the compound of formula (I) (0 μM, 10 μM, 30 μM, and 90 μM) for 72 h; Figure 7E, 7F, 7G, and 7H are representative cell apoptosis plots of NCI-H460 cells treated with different concentrations of the compound of formula (I) (0 μM, 10 μM, 30 μM, and 90 μM) for 72 h; Figure 7I shows the proportion of total apoptotic cells of A549 cells after being co-cultured with the compound of formula (I) at different concentrations; Figure 7G shows the proportion of total apoptotic cells of NCI-H460 cells after being co-cultured with the compound of formula (I) at different concentrations; **: P<0.01.

Figure 8 shows the Western blot (A) and grayscale analysis (B) results of NCI-N87 tumor tissue proteins. In an NCI-N87 (3D_PN154004) transplanted tumor model in a nude mouse, the solution of compound of formula (I) (2mg/kg) downregulated p-VEGFR2 (NS), downregulated VEGFR2 (P<0.05) and VEGFA (P<0.05), downregulated β-catenin (P<0.05) and CyclinD1 (NS), upregulated PCNA (NS), upregulated cell cycle regulatory protein P21 (NS), downregulated pro-apoptotic Bax (P<0.01) and anti-apoptotic protein Survivin (P<0.05). "NS" indicates that there was no statistically difference between the drug administration group and the blank solvent group (P>0.05); "*" indicates that there was a significant difference between the drug administration group and the blank solvent group (P<0.05); "**" indicates that there was a highly significant difference between the drug administration group and the blank solvent group (P<0.01).

Figure 9 shows that the glomeruli and renal tubules of mice in the normally fed blank control group were basically undamaged, and only some areas of the renal tubule walls were thinned and slightly damaged (the picture was PAS stained, and magnified 20 times).

Figure 10 shows the severe loss of renal tubular epithelium after the mice in the model group was administered with 10 mg/kg of cisplatin: the brush border of cells was lost, and a large number of protein casts appeared (the picture was HE stained, and magnified 20 times).

Figure 11 shows that mice in the positive control group only developed slight renal tubular damage and blurred renal tubular epithelial edges after being administered with cisplatin (10 mg/kg) and amifostine (50 mg/kg, intraperitoneally injected half an hour before the administration of cisplatin) (the picture was HE stained, and magnified 40 times).

Figure 12 shows that mice in the assay group developed severe renal tubular damage, epithelial cell necrosis and slight protein casts after being administered with cisplatin (10 mg/kg) and R01 (6 mg) (the picture was HE stained, and magnified 40 times).

Figure 13 shows that mice in the assay group developed visible epithelial cell necrosis and a certain degree of renal tubular damage after being administered with cisplatin (10 mg/kg) and R01 (18 mg) (the picture was HE stained, and magnified 40 times).

Figure 14 shows that mice in the assay group developed slight kidney damage after being administered with cisplatin (10 mg/kg) and R01 (36 mg), including renal tubule lumen enlargement, epithelial cell necrosis, etc. (the picture was HE stained, and magnified 40 times).

## DETAILED DESCRIPTION

In a first aspect, the present disclosure provides a compound of formula (I), or a solvate, a hydrate, a polymorph, a prodrug or an isotopic variant thereof, or a mixture thereof.

In a second aspect, the present disclosure provides a pharmaceutical composition, comprising the compound of formula (I), and optionally pharmaceutically acceptable excipient(s).

In a third aspect, the present disclosure provides a pharmaceutical composition comprising the compound of formula (I) and pharmaceutically acceptable excipient(s), and optionally other therapeutic agent(s).

In a fourth aspect, the present disclosure provides a kit, comprising the compound of formula (I), other therapeutic agent(s), and pharmaceutically acceptable carrier(s), adjuvant(s), or vehicle(s).

In a fifth aspect, the present disclosure provides use of the compound of formula (I) in the manufacture of a medicament for the treatment and/or prevention of a cancer in a subject.

In a sixth aspect, the present disclosure provides a method of treating and/or preventing a cancer in a subject, comprising administering the compound of formula (I) or a composition of the present disclosure to the subject.

In a seventh aspect, the present disclosure provides the compound of formula (I) or a composition of the present disclosure, for use in the treatment and/or prevention of a cancer in a subject.

In an eighth aspect, the present disclosure provides a combination, comprising the compound of formula (I), or a solvate, a hydrate, a polymorph, a prodrug or an isotopic variant thereof, and a platinum-based drug.

In a ninth aspect, the present disclosure provides a pharmaceutical composition, comprising the combination of the eighth aspect and pharmaceutically acceptable carrier(s), diluent(s) or excipient(s).

In a tenth aspect, the present disclosure provides use of the combination of the eighth aspect in the manufacture

of a medicament for the treatment and/or prevention of a cancer in a subject.

In an eleventh aspect, the present disclosure provides a pharmaceutical product, comprising the compound of formula (I), or a solvate, a hydrate, a polymorph, a prodrug or an isotopic variant thereof, and a platinum-based drug, as a combination formulation for simultaneous, sequential or separate use in treatment.

In a twelfth aspect, the present disclosure provides a method of treating and/or preventing a cancer, comprising administering to a subject or patient the compound of formula (I), or a solvate, a hydrate, a polymorph, a prodrug or an isotopic variant thereof, and a platinum-based drug simultaneously, sequentially, or separately.

In a thirteenth aspect, the present disclosure provides use of the compound of formula (I), or a solvate, a hydrate, a polymorph, a prodrug or an isotopic variant thereof, in the manufacture of a medicament for the treatment and/or prevention of a cancer, wherein the treatment includes administering to a patient or subject the compound of formula (I), or a solvate, a hydrate, a polymorph, a prodrug or an isotopic variant thereof, and a platinum-based drug simultaneously, sequentially, or separately.

In a fourteenth aspect, the present disclosure provides use of the compound of formula (I), or a solvate, a hydrate, a polymorph, a prodrug or an isotopic variant thereof, and a platinum-based drug in the manufacture of a medicament for the treatment and/or prevention of a cancer in a subject.

In a fifteenth aspect, the present disclosure provides use of the compound of formula (I), or a solvate, a hydrate, a polymorph, a prodrug or an isotopic variant thereof, in the manufacture of a medicament for the treatment and/or prevention of a cancer in a subject, wherein the medicament is used in a combination therapy with a platinum-based drug.

In a sixteenth aspect, the present disclosure provides use of a platinum-based drug in the manufacture of a medicament for the treatment and/or prevention of a cancer in a subject, wherein the medicament is used in a combination therapy with the compound of formula (I), or a solvate, a hydrate, a polymorph, a prodrug or an isotopic variant thereof.

In a seventeenth aspect, the present disclosure provides use of the compound of formula (I), or a solvate, a hydrate, a polymorph, a prodrug or an isotopic variant thereof, in the manufacture of a medicament for alleviation of acute kidney damage induced by platinum-based drug(s) in a subject.

In an eighteenth aspect, the present disclosure provides a method of alleviating acute kidney damage induced by platinum-based drug(s) in a subject, comprising administering the compound of formula (I), or a solvate, a hydrate, a polymorph, a prodrug or an isotopic variant thereof, simultaneously, sequentially or separately with the administration of the platinum-based drug(s).

In the above aspects, the platinum-based drug is selected from cisplatin, carboplatin, nedaplatin, oxaliplatin, and loplatin.

In the above aspects, the cancer is selected from lung cancer, gastric cancer, esophageal cancer, and colorectal cancer.

Other objects and advantages of the present disclosure will be apparent to those skilled in the art from the subsequent specific embodiments, examples and claims.

## Definitions

The term "cancer" includes, but is not limited to, the following cancers: gastric cancer, lung cancer, esophageal cancer, and colorectal cancer. More specifically, the cancer includes, but is not limited to, HER2-overexpressing metastatic gastric adenocarcinoma or gastroesophageal junction adenocarcinoma, non-small cell lung cancer with sensitive mutations in the epidermal growth factor receptor (EGFR) gene, locally advanced or metastatic squamous histology type non-small cell lung cancer that progresses during or after platinum-containing chemotherapy, etc.

The term "treating" as used herein relates to reversing, alleviating or inhibiting the progression or prevention of the disorders or conditions to which the term applies, or of one or more symptoms of such disorders or conditions. The noun "treatment" as used herein relates to the action of treating, which is a verb, and the latter is as just defined.

"Subjects" to which administration is contemplated include, but are not limited to, humans (e.g., males or females of any age group, e.g., paediatric subjects (e.g., infants, children, adolescents) or adult subjects (e.g., young adults, middle-aged adults or older adults)) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "humam", "patient" and "subject" can be used interchangeably herein.

"Disease", "disorder", and "condition" can be used interchangeably herein.

Unless indicated otherwise, the term "treatment" as used herein includes the effect on a subject who is suffering from a particular disease, disorder, or condition, which reduces the severity of the disease, disorder, or condition, or delays or slows the progression of the disease, disorder or condition ("therapeutic treatment"). The term also includes the effect that occurs before the subject begins to suffer from a specific disease, disorder or condition ("prophylactic treatment").

Generally, the "effective amount" of a compound refers to an amount sufficient to elicit a target biological

response. As understood by those skilled in the art, the effective amount of the compound of the present disclosure (i.e. compound of formula (I)) can vary depending on the following factors, such as the desired biological endpoint, the pharmacokinetics of the compound, the diseases being treated, the mode of administration, and the age, health status and symptoms of the subjects. The effective amount includes therapeutically effective amount and prophylactically effective amount.

Unless indicated otherwise, the "therapeutically effective amount" of the compound as used herein is an amount sufficient to provide therapeutic benefits in the course of treating a disease, disorder or condition, or to delay or minimize one or more symptoms associated with the disease, disorder or condition. The therapeutically effective amount of a compound refers to the amount of the therapeutic agent that, when used alone or in combination with other therapies, provides a therapeutic benefit in the treatment of a disease, disorder or condition. The term "therapeutically effective amount" can include an amount that improves the overall treatment, reduces or avoids the symptoms or causes of the disease or condition, or enhances the therapeutic effect of other therapeutic agents.

Unless indicated otherwise, the "prophylactically effective amount" of the compound as used herein is an amount sufficient to prevent a disease, disorder or condition, or an amount sufficient to prevent one or more symptoms associated with a disease, disorder or condition, or an amount sufficient to prevent the recurrence of a disease, disorder or condition. The prophylactically effective amount of a compound refers to the amount of a therapeutic agent that, when used alone or in combination with other agents, provides a prophylactic benefit in the prevention of a disease, disorder or condition. The term "prophylactically effective amount" can include an amount that improves the overall prevention, or an amount that enhances the prophylactic effect of other preventive agents.

"Combination" and related terms refer to the simultaneous or sequential administration of the compound of the present disclosure and other therapeutic agents. For example, the compound of the present disclosure can be administered simultaneously or sequentially in separate unit dosage with other therapeutic agents, or simultaneously in a single unit dosage with other therapeutic agents.

It will be understood by those skilled in the art that the organic compounds can form complexes with solvents in which they are reacted or from which they are precipitated or crystallized. These complexes are known as "solvates". Where the solvent is water, the complex is known as "hydrate". The present disclosure encompasses all solvates of the compound of the present disclosure.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compound described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula $R \cdot x\ H_2O$, wherein R is the compound, and x is a number greater than 0. Given compounds can form more than one type of hydrates, including, for example, monohydrates (x is 1), lower hydrates (x is a number greater than 0 and smaller than 1, for example, hemihydrates ($R \cdot 0.5\ H_2O$)) and polyhydrates (x is a number greater than 1, for example, dihydrates ($R \cdot 2\ H_2O$) and hexahydrates ($R \cdot 6\ H_2O$)).

The compound of the present disclosure may be in an amorphous or a crystalline form (polymorph). Furthermore, the compound of the present disclosure may exist in one or more crystalline forms. Therefore, the present disclosure includes all amorphous or crystalline forms of the compound of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes (isotopic variants), which are equivalent to that described in formula (I), but one or more atoms are replaced by atoms having an atom mass or mass number that are different from that of atoms that are common in nature. Examples of isotopes which may be introduced into the compound of the disclosure include isotopes of hydrogen, carbon, nitrogen, oxygen, phosphorus, sulfur, fluorine and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain

isotope-labeled compounds of the present disclosure, such as those incorporating radioactive isotopes (e.g., $^3H$ and $^{14}C$), can be used for the measurement of the distribution of drug and/or substrate in tissue. Tritium, which is $^3H$ and carbon-14, which is $^{14}C$ isotope, are still alternative, because they are easy to prepare and detect. Furthermore, replaced by heavier isotopes, such as deuterium, which is $^2H$, may provide therapeutic benefits due to the higher metabolic stability, such as prolonging the half-life in vivo or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted into an active form that has medical effects *in vivo* by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which are incorporated herein by reference.

The prodrugs are any covalently bonded compound of the present disclosure, which release a parent compound in the body when administered to a patient. Prodrugs are typically prepared by modifying functional groups in such a way that the modifications can be cleaved either by routine manipulation or decomposition in the body to yield a parent compound. Prodrugs include, for example, the compound of the present disclosure in which a hydroxyl, amino or sulfhydryl group is bonded to any group, wherein the bond can be cleaved to form the hydroxyl, amino or sulfhydryl group when the compound is administered to a patient. Thus, representative examples of prodrugs include (but are not limited to) the acetate/acetamide, formate/formamide and benzoate/benzamide derivatives of the hydroxyl, amino or sulfhydryl functional group of the compound of formula (I). Furthermore, in the case of carboxylic acid (-COOH), esters such as methyl esters and ethyl esters, etc. can be employed. The ester may be active in their own and/or hydrolyzable under *in vivo* conditions in the human body. Suitable pharmaceutically acceptable *in vivo* hydrolysable ester groups include those groups that can be readily broken down in the human body to release the parent acids or salts thereof.

The present disclosure also provides a pharmaceutical formulation, comprising a therapeutically effective amount of the compound of formula (I), and pharmaceutically acceptable carrier(s), diluent(s) or excipient(s). All of these forms belong to the present disclosure.

The term "manufacture of a medicament" as used herein includes use of the ingredients of the present disclosure directly as a medicament in addition to use in any stage of the manufacture of a medicament.

The term "combination therapy" as used herein refers to a therapy, in which the compound of formula (I), or a solvate, a hydrate, a polymorph, a prodrug or an isotopic variant thereof, and a platinum-based drug were administered sequentially, if not simultaneously, within a time limit, so that they can both have a therapeutic effect within the same time limit.

As described above, one aspect of the present disclosure relates to a pharmaceutical product, comprising the compound of formula (I), or a solvate, a hydrate, a polymorph, a prodrug or an isotopic variant thereof, and a platinum-based drug, as a combination formulation for simultaneous, sequential or separate use in treatment.

As used herein, "simultaneously" refers to the administration of two agents at the same time, while the term "combination" refers to the administration of two agents "sequentially", if not simultaneously, within a time limit, so that they are both suitable to have a therapeutic effect within the same time limit. Thus, "sequential" administration may allow the administration of one agent 5 minutes, 10 minutes, or approximately a few hours after another, as long as the circulating half-life of the first administered agent allows both to be present in therapeutically effective amounts. The delay between the administration of ingredients varies depending on the exact nature of the ingredients, their interactions and their respective half-lives.

Compared with "combination" or "sequentially", "separately" as used herein refers to an obvious interval between the administration of one agent and another, that is, when the second agent is administered, there is no longer therapeutically effective amount of the first administered agent in the bloodstream.

In another alternative embodiment, compared with administering an individual component, a subtherapeutic amount of the compound of formula (I), or a solvate, a hydrate, a polymorph, a prodrug or an isotopic variant thereof, and a subtherapeutic amount of a platinum-based drug are administered, respectively. In other words, if not administered in combination, the compound of formula (I), or a solvate, a hydrate, a polymorph, a prodrug or an isotopic variant thereof and a platinum-based drug are not administered in therapeutically effective amounts.

Alternatively, the compound of formula (I), or a solvate, a hydrate, a polymorph, a prodrug or an isotopic variant thereof, and a platinum-based drug interact in a synergistic manner. The term "synergistic" as used herein refers to the ability of the compound of formula (I), or a solvate, a hydrate, a polymorph, a prodrug or an isotopic variant thereof, and a platinum-based drug when used in combination to produce a greater effect than the expected effect obtained by sum of the individual effects of the two components. Advantageously, the synergistic interactions may allow each component

to be administered to patients at a lower dosage, thereby reducing the toxicity of chemotherapy while producing and/or maintaining the same therapeutic effect. Thus, in a still alternative embodiment, each component may be administered in a subtherapeutic amount.

## Pharmaceutical compositions and kits

In another aspect, the present disclosure provides a pharmaceutical composition, comprising a compound of the present disclosure (also referred to as the "active ingredient") and pharmaceutically acceptable excipient(s). In some embodiments, the pharmaceutical composition comprises an effective amount of the compound of the present disclosure. In some embodiments, the pharmaceutical composition comprises a therapeutically effective amount of the compound of the present disclosure. In some embodiments, the pharmaceutical composition comprises a prophylactically effective amount of the compound of the present disclosure.

Pharmaceutically acceptable excipients for use in the present disclosure refer to the nontoxic carriers, adjuvants or vehicles, which do not destroy the pharmacological activity of the compounds formulated together. Pharmaceutically acceptable carriers, adjuvants, or vehicles that can be used in the compositions of the present disclosure include (but are not limited to) ion exchangers, alumina, aluminum stearate, lecithin, serum proteins (such as human serum proteins), buffer substances (such as phosphate), glycine, sorbic acid, potassium sorbate, mixture of partial glycerides of saturated plant fatty acids, water, salts or electrolytes (such as protamine sulfate), disodium hydrogen phosphate, potassium hydrogen phosphate, sodium chloride, zinc salts, silica gel, magnesium trisilicate, polyvinyl pyrrolidone, cellulose-based substance, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). The kits provided may include a compound of the present disclosure, other therapeutic agent(s), and a first and a second containers (e.g., vials, ampoules, bottles, syringes, and/or dispersible packages or other suitable containers) containing the compound of the present disclosure and other therapeutic agent(s). In some embodiments, the provided kits can also optionally include a third container containing a pharmaceutically acceptable excipient for diluting or suspending the compound of the present disclosure and/or other therapeutic agent(s). In some embodiments, the compound of the present disclosure provided in the first container and other therapeutic agent(s) provided in the second container are combined to form a unit dosage form.

## Administration

The pharmaceutical composition provided by the present disclosure can be administered by a variety of routes including, but not limited to, oral administration, parenteral administration, inhalation administration, topical administration, rectal administration, nasal administration, buccal administration, vaginal administration, administration by implant or other means of administration. For example, parenteral administration as used herein includes subcutaneous administration, intradermal administration, intravenous administration, intramuscular administration, intra-articular administration, intra-arterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the route of administration selected, the actual compound administered, the age, weight and response of the individual patient, the severity of the patient’s symptoms, etc.

When used to prevent the conditions described in the present disclosure, the compounds provided herein will be administered to a subject at risk of developing the conditions, typically based on the physician’s recommendation and administered under the supervision of the physician, at the dosage level described above. Subjects at risk of developing the particular conditions generally include those who have a family history of the conditions, or those who have been identified by genetic testing or screening to be particularly susceptible to developing the conditions.

The pharmaceutical compositions provided herein can also be administered chronically ("chronic administration"). Chronic administration refers to the administration of a compound or pharmaceutical composition thereof for a long period of time, for example, 3 months, 6 months, 1 year, 2 years, 3 years, 5 years, etc., or can be continuously administered indefinitely, for example, for the rest of the subject’s life. In some embodiments, the chronic administration is intended to provide a constant level of the said compound in the blood over a long period of time, for example, within the therapeutic window.

Pharmaceutical compositions of the present disclosure can be further delivered using various dosing methods. For example, in some embodiments, pharmaceutical compositions can be administered by bolus injection, for example, to increase the concentration of the compound in the blood to an effective level. The bolus dose depends on the desired systemic level of the active ingredient throughout the body, for example, intramuscular or subcutaneous bolus dose

allows a slow release of the active ingredient, while the bolus that is delivered directly to the vein (e.g., via IV intravenous drip) allows a much faster delivery which quickly raises the concentration of the active ingredient in the blood to an effective level. In other embodiments, pharmaceutical compositions can be administered in a form of continuous infusion, for example, via IV intravenous drip, thereby providing a steady state concentration of the active ingredient in the subject's body. Moreover, in other embodiments, a bolus dose of the pharmaceutical compositions can be administered first, followed by continuous infusion.

The compositions for oral administration can be in the form of bulk liquid solution or suspension or bulk powder. More commonly, however, in order to facilitate the precise dosing, the compositions are provided in unit dosage form. The term "unit dosage form" refers to physical discrete units suitable as unitary dosages for human patients and other mammals, each unit containing a predetermined quantity of active ingredient calculated to produce the desired therapeutic effects with suitable pharmaceutical excipient(s). Typical unit dosage forms include prefilled, pre-measured ampoules or syringes of the liquid compositions, or pills, tablets, capsules, etc. in the case of solid compositions. In such compositions, the said compound generally will be the minor component (about 0.1 to about 50% by weight, or alternatively about 1 to about 40% by weight), with the remainder being various carriers or excipients and processing aids useful for forming the desired dosing form.

For oral dosage, a representative scheme is one to five, especially two to four, and typically three oral doses per day. Using these dosing patterns, each dose provides from about 0.01 to about 100 mg/kg of the compound of the present disclosure, with alternative doses each providing from about 0.1 to about 10 mg/kg, and especially from about 0.5 to about 2 mg/kg.

The injection dosage level ranges from about 0.1 mg/kg/hr to at least 10 mg/kg/hr, all for from about 1 to about 120 hours, especially from 24 to 96 hours. In order to achieve a sufficient level of steady state, a preloading bolus of from about 0.1 mg/kg to about 10 mg/kg or more can also be administered. For human patients of 40 to 80 kg, the maximum total dosage should not exceed approximately 2 g/day.

Liquid forms suitable for oral administration may include suitable aqueous or nonaqueous carriers, buffers, suspending agents and dispersants, coloring agents, flavouring agents, etc. Solid forms may include, for example, any of the following components, or compounds having the similar properties: binders, for example, microcrystalline cellulose, tragacanth gum or gelatin; excipients, for example, starch or lactose; disintegrants, for example, alginic acid, Primogel or corn starch; lubricants, for example, magnesium stearate; glidants, for example, colloidal silica; sweeteners, for example, sucrose or saccharin; or flavouring agents, for example, peppermint, methyl salicylate or orange flavouring.

Injectable compositions are typically based on the injectable sterile saline or phosphate-buffered saline, or other injectable excipients known in the art. As previously mentioned, in such compositions, the active ingredients will typically be the minor component, often from about 0.05 to 10% by weight, with the remainder being injectable excipients, etc.

The transdermal compositions are typically formulated as topical ointments or creams containing the active ingredients. When formulated as an ointment, the active ingredients are typically combined with paraffin or water miscible ointment base. Alternatively, the active ingredients can be formulated as a cream with, for example, oil-in-water cream base. Such transdermal formulations are well-known in the art and generally include other ingredients for enhancing stable skin penetration of the active ingredients or the formulations. All such known transdermal formulations and components are included within the scope of the present disclosure.

The compound of the present disclosure may also be administered by transdermal devices. Thus, transdermal administration can be accomplished using a patch either of reservoir or porous membrane type, or of a plurality of solid substrates.

The above components of the compositions for oral administration, injection or topical administration are only representative. Other materials and processing techniques, etc., are described in the Section 8 of Remington's Pharmaceutical Sciences, 17th edition, 1985, Mack Publishing Company, Easton, Pennsylvania, which is incorporated herein by reference.

The compound of the present disclosure may also be administered in a sustained release form or from a sustained release delivery system. Description of the representative sustained release materials can be found in Remington's Pharmaceutical Sciences.

## Treatment

Therefore, the compound of the present disclosure has value as an anti-tumor agent. In particular, the compound of the present disclosure has value as an anti-proliferative, apoptotic and/or anti-invasive agent in the containment and/or treatment of solid and/or liquid tumor diseases. In particular, the compound of the present disclosure is expected to be useful in preventing or treating gastric cancer, lung cancer, and the like.

Anti-cancer effects useful for treating cancer in patients include, but are not limited to, anti-tumor effect, response rate, time of disease progression, and survival rate. The anti-tumor effects of the treatment methods of the present

disclosure include, but are not limited to, inhibition of tumor growth, delay of tumor growth, tumor regression, tumor shrinkage, prolonged tumor regeneration after the cessation of treatment, and slowing of disease progression. The anti-cancer effects include preventive treatment as well as treatment of the existing diseases.

The effective amount of the compound of the present disclosure is generally at an average daily dosage of from 0.01 mg to 50 mg of the compound per kilogram of patient's body weight, alternatively from 0.1 mg to 25 mg of the compound per kilogram of patient's body weight, administered in a single or multiple doses. Generally, the compound of the present disclosure may be administered to a patient in need of such treatment in a daily dosage range from about 1 mg to about 3500 mg per patient, alternatively from 10 mg to 1000 mg per patient. For example, the daily dosage for each patient may be 10, 20, 30, 40, 50, 60, 70, 80, 90, 100, 120, 150, 160, 180, 200, 240, 250, 300, 350, 360, 400, 500, 600, 700, 800, 900 or 1000 mg. It can be administered one or more times daily, weekly (or at intervals of several days) or on an intermittent schedule. For example, the compound can be administered one or more times per day on a weekly basis (e.g., every Monday), continually or for several weeks, such as 4-10 weeks. Alternatively, the compound may be administered daily for several days (e.g., 2-10 days) and then the compound is not administered for several days (e.g., 1-30 days), and the cycle may be repeated continuously or for a given number of times, e.g., 4-10 cycles. For example, the compound of the present invention may be administered daily for 5 days, followed by a 9-day break, then administered daily for 5 days, followed by a 9-day break, and so on, repeating this cycle continuously or a total of 4-10 times.

The platinum-based drugs used in combination with the compound of formula (I) of the present disclosure, or a solvate, a hydrate, a polymorph, a prodrug or an isotope variant thereof, are recommended to be at the common dosage or recommended dosage in clinical practice. For example, the common dosage of cisplatin is 50 to 100 mg/$m^2$, or an intravenous infusion of 15 to 20 mg/$m^2$ daily is continued for 5 days, and both were administrated repeatedly for 3 to 4 weeks. For carboplatin, the recommended dosage for patients with normal renal function is 400 mg/$m^2$; for patients with risk factors, it is recommended that the initial dosage is reduced by 20 to 25%; for patients over 65 years old, the initial dosage and the subsequent therapeutic dosages should be adjusted according to the patient's physical conditions. For nedaplatin, 80 to 100 mg/$m^2$ is given per dose, with an interval of 3 to 4 weeks before the next course of treatment. For oxaliplatin, the recommended dosage is 130 mg/$m^2$ as a single dose, which is administered every 3 weeks (21 days) in the absence of major toxicity, or 85 mg/$m^2$, which is repeated every 2 weeks. For lobaplatin, the recommended dosage is 50 mg/$m^2$ as a single dose, and a complete recovery from the blood toxicity or other clinical side effects is required before the next use. The recommended interval of administration is 3 weeks. If the recovery from side effects is slow, the interval of administration can be extended.

When the compound of formula (I) of the present disclosure, or a solvate, a hydrate, a polymorph, a prodrug or an isotopic variant thereof, is used in combination with a platinum-based drug, compared with administering the individual components, a subtherapeutic amount of the compound of formula (I), or a solvate, a hydrate, a polymorph, a prodrug or an isotopic variant thereof, and a subtherapeutic amount of a platinum-based drug may also be administered, respectively.

## EXAMPLES

The materials or reagents used herein are commercially available or prepared by synthetic methods commonly known in the art.

### Example 1: Synthesis of the compound of formula (I)

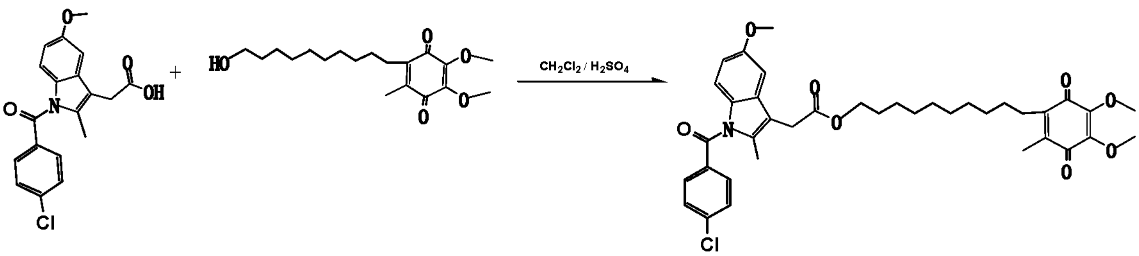


Anhydrous dichloromethane (40ml), 6-(10-hydroxydecyl)-2,3-dimethoxy-5-methyl-1,4-benzoquinone (3.38g, 0.01mol), and concentrated sulfuric acid (0.6ml) were placed in a round-bottom flask, and the mixture was stirred to give a solution. Then, 4A molecular sieve (2.5g) and 2-methyl-1-(4-chlorobenzoyl)-5-methoxy-1H-indole-3-acetic acid (3.57g, 0.01mol) were added, refluxed for 10 hours, and then filtered. The filtrate was washed with an appropriate amount of

sodium carbonate aqueous solution and saturated brine, respectively, then washed with water until neutral, dried over anhydrous sodium sulfate, and concentrated to give a black crude product, which was purified by column chromatography to give 3.98 g of brown solid, with a yield of 59.7% and an HPLC purity of 97.9%.

$^1$H-NMR (400 MHz, d$^6$DMSO): δ 7.64-7.67(d, 2H), 7.45-7.48(d, 2H), 6.96 (s, 1H), 6.85-6.87(d, 1H), 6.65-6.67(d, 1H), 4.07-4.11(t, 2H), 3.98 (s, 6H), 3.83 (s, 3H), 3.66 (s, 2H), 2.42-2.46(t, 2H), 2.39 (s, 3H), 2.0 (s, 3H), 1.58-1.64(m, 2H), 1.24-1.40(m, 14H).

ESI-MS: 678.2 (M+1), 700.2 (M+23).

**Example 2: Study on the effects of the compound of formula (I) on tumor cell proliferation, tumor cell cycle and apoptosis**

**1. Main instruments and equipment**

| Main instrument | Manufacturer |
|---|---|
| Carbon dioxide incubator | ESCO |
| Biological safety cabinet | Qingdao Haier Biomedical Co., Ltd. |
| Vacuum aspirator | Jiangsu Yuyue Medical Instruments Co., Ltd. |
| Inverted fluorescence microscope | Leica |
| Inverted biological microscope AE2000 | Motic |
| Ice maker | Scotsman, Italy |
| Electric blast drying oven | Shanghai Yiheng Scientific Instruments Co., Ltd. |
| -80°C ultra-low temperature storage freezer | Qingdao Haier Biomedical Co., Ltd. |
| 4°C/-20 °C refrigerator | Hisense Rongsheng (Guangdong) (Refrigerator Co., Ltd. |
| Electric thermostatic water tank | Shanghai Yiheng Scientific Instruments Co., Ltd. |
| Low speed automatic balancing centrifuge | Beijing Jingli Centrifuge Co., Ltd. |
| Small high speed refrigerated centrifuge | Eppendorf, Germany |
| Benchtop high speed refrigerated centrifuge | Shanghai Lishen Scientific Instruments Co., Ltd. |
| Vertical shaker | Crystal, USA |
| Precision electronic balance | Shanghai Jingke Scientific Instrument Co., Ltd. |
| Handheld homogenizer | Dremel, USA |
| Handheld centrifuge | SCILOGEX |
| Adjustable mixer | SCILOGEX |
| Ultrapure water system | Hong Kong Heal Force Bio-Meditech Holdings Limited |
| Constant temperature oscillator | Crystal, USA |
| Flow cytometers | BD Biosciences, USA |
| NanoDrop2000C ultra-trace spectrophotometer | Thermo Fisher Scientific, USA |

**2. Test samples and cell lines**

Human lung cancer cell lines A549 and NCI-H460 were both from the cell bank of the Oncology Department Laboratory of Tongji Hospital.

**3. Main assay reagents**

| Main reagents | Source |
|---|---|
| Fetal bovine serum (FBS) | Gibco, USA |
| RPMI-1640 medium | Hyclone, USA |
| DMEM medium | Hyclone, USA |

(continued)

| Main reagents | Source |
|---|---|
| PBS | Hyclone, USA |
| 0.25% Trypsin-EDTA | Gibco, USA |
| 100×Pen Strep Glutamine | Gibco, USA |
| 75% Alcohol | Zhongbei Alcohol Packaging Factory in Wuchang, Wuhan |
| Dimethyl Sulfoxide for cell culture | PanReac AppliChem GmbH, Germany |
| Tween 20 | Biosharp, Hefei |
| TEMED | Biosharp, Hefei |
| Glycine | Biofroxx, Germany |
| Tris-base | Biosharp, Hefei |
| Sodium chloride | Sinopharm Chemical Reagent Co., Ltd. |
| Potassium chloride | Sinopharm Chemical Reagent Co., Ltd. |
| Methanol | Sinopharm Chemical Reagent Co., Ltd. |
| DTT | Wuhan Promoter Biology Co., Ltd. |
| PMSF | Wuhan Promoter Biology Co., Ltd. |
| Albumin bovine v | Biosharp, Hefei |
| Tris-HCL | Biofroxx, Germany |
| HEPES | Biosharp, Hefei |
| EDTA | Biosharp, Hefei |
| EGTA | Biosharp, Hefei |
| Triton X-100 | Biosharp, Hefei |
| Isopropyl alcohol | Sinopharm Chemical Reagent Co., Ltd. |
| Chloroform | Sinopharm Chemical Reagent Co., Ltd. |
| Anhydrous ethanol | Sinopharm Chemical Reagent Co., Ltd. |
| Crystal violet | Wuhan Sevier Biotechnology Co., Ltd. |
| Annexin V-FITC/PI apoptosis detection kit | BD Biosciences, USA |
| Cell Counting Kit-8 | Wuhan Promoter Biology Co., Ltd. |
| Cell-Light EDU Apollo567 Kit | Shanghai Xinrui Biotechnology Co., Ltd. |
| Cell cycle detection kit | Wuhan Promoter Biology Co., Ltd. |
| Sterile DEPC water | Wuhan Promoter Biology Co., Ltd. |

**4.** Related solutions included 10% BSA (1g of albumin bovine v was diluted to 10ml with ultrapure water), TBST (50ml of 10×TBS and 500μl of Tween 20 were diluted to 500ml with ultrapure water), cell cryopreservation solution (prepared from 28ml of RPMI-1640 medium, 8ml of FBS and 4ml of DMSO, and stored at 4 °C), and cell lysis solution (1ml of 500μM HEPES, 500μl of 3M sodium chloride, 20μl of 500μM EDTA, 20μl of 500μM EGTA, 250μl of 20% Triton X-100, and 7.7ml of $ddH_2O$ were mixed, and the mixture was divided into 1ml in each tube, and stored at -20 °C. 1M DTT (added when being used) 1: 1000, 100μM PMSF (added when being used) 1: 100, and 25×Cocktail (added when being used) 1:25).

Storage solution of the compound of formula (I): The compound of formula (I) is easily soluble in organic solvents, so DMSO and isopropyl alcohol in a ratio of 3:7 were selected as the solvents. The containers with DMSO and isopropyl alcohol were opened in a biological safety cabinet. A 15ml centrifuge tube was taken, and 3ml of DMSO and 7ml of isopropyl alcohol were added to make a mixed solvent. The molecular weight of the compound of formula (I) is 678.22g/mol. 40.69 mg of an original drug of the compound of formula (I) was weighed, and 4 ml of the mixed solvent was added. The mixture was mixed well by pipetting. The drug storage solution had a concentration of 15000 μM according to a conversion, and was divided into 1.5 ml EP tubes, which were marked with name, concentration and time with a marker, and stored at 4°C. The solution was diluted according to the required concentration when being used.

**5. Assay method**

**Cell culture**

(1) Cell recovery

The electric thermostatic water tank was turned on in advance, and the temperature was set at 37 °C. When the temperature rised to the set temperature, a cell cryopreservation tube was taken out from the -80 °C freezer and placed in the 37 °C water tank. The cryopreservation tube was gently shaked to melt the cells quickly. The cell cryopreservation tube was quickly put into a low-speed centrifuge with a set speed of 1200 rpm, and centrifuged for 5 minutes. After the centrifugation, 75% alcohol was sprayed on the outside of the cryopreservation tube. The cryopreservation tube was opened in a biological safety cabinet, and the supernatant was aspirated with a vacuum aspirator. 1ml of fresh complete culture medium was pipetted into the cryopreservation tube using a pipette to resuspend the cells. After mixing by pipetting, the cell suspension was transferred to a T25 cell culture flask or a culture dish with a diameter of 6 cm, and 3 ml of fresh culture medium was then added. The cells were cultured in an incubator at 37 °C with 5% $CO_2$. The adhesion and growth status of the recovered cells were in need to be observed the next day, and the cell culture medium was changed if necessary.

(2) Cell culture medium replacement

The complete culture medium, PBS, etc. were placed in a 37 °C water bath to be preheated for a few minutes in advance, and then were placed in a biological safety cabinet after being sprayed on the outside with 75% alcohol. The 6cm culture dish containing cells were taken out from the incubator, and cell status was observed under an inverted biological microscope. The vacuum aspirator was turned on, and the culture medium in the culture dish was aspirated. The culture dish was washed 1 to 2 times with PBS, and the PBS was discarded. 3 ml of complete culture medium was added to the culture dish, which was placed in an incubator at 37 °C with 5% $CO_2$ to continue the culturing.

(3) Cell passaging and cryopreservation

The cell growth status was observed under an inverted biological microscope. Cell passaging was required when the cells grew to 80% to 90% of the bottom area of the culture flask. The culture flask was placed in a biological safety cabinet. The vacuum aspirator was turned on, and the old culture medium was aspirated with the vacuum aspirator. An appropriate amount of PBS was gently added with a pipette to rinse the cells 1 to 2 times, and the PBS was discarded. An appropriate amount of trypsin was added to the bottom of the culture flask, and the culture flask was gently shaked to evenly cover the cell surface with trypsin, and placed back to the 37°C incubator. Note that the digestion time of different cells is slightly different, generally from 2 to 3 minutes. When the cells were observed under the microscope to be dissociated, round and translucent, and detached from the culture flask to flow, 5 to 8 times the trypsin volume of complete culture medium was added to terminate the digestion. The digested cells were slowly pipetted with a pipett to disperse the digested cells into a single cell suspension, which was transferred to a 4ml centrifuge tube to centrifuge at 1500rpm for 5min. The supernatant was discarded, and complete culture medium was added to resuspend the cells. An appropriate amount of the cell suspension was retained in the culture flask as needed, and the remaining cell suspension was added to a cryovial. Complete culture medium was added to the culture flask to 3 ml to continue the culturing. A cryopreservation solution at a volume ratio of 1:1 was added to the cryovial, which was then placed in a cryopreservation box in the -80 °C freezer, and the update was recorded on a cell sheet.

**CCK-8 assay**

(1) Cells in the logarithmic growth phase were taken and digested to make a cell suspension. 20 μl of the cell suspension was taken with a pipette, and added to a CountStar disposable cell counting plate for cell counting, and the counting results were recorded. The cell concentration was adjusted to 30,000 to 40,000 cells/ml (note: different cells have different growth rates, so the number of cells in each well was slightly different).

(2) A 96-well plate was taken. Since the wells in the outermost circle of the culture plate were easy to evaporate and dry, causing inaccurate volumes, and increasing errors, 100 μl of PBS was added to the wells in the outermost circle, which were not used as assay wells.

(3) The adjusted cell suspension was added to the 96-well plate, with 100 μl in each well, resulting approximately 3000 to 4000 cells in each well. The plate was placed in a 37 °C incubator under the condition of 5% $CO_2$ for cell

culture, and the drug was added when the cells adhered to the wall.

(4) Drug dilution. The concentration gradient was set as: 0μM, 10μM, 50μM, 100μM, and 200μM, with 3 duplicates for each concentration. In addition, blank control wells containing only culture medium and drug without cells were also set.

(5) The plate was incubated in the incubator for 24h, 48h and 72h, respectively. After reaching the set time point, the 96-well plate was taken out from the incubator, and 10 μl of CCK-8 reagent was added to each well, taking care to avoid bubbles. The plate was shaked to mix evenly, and put back into the incubator to culture for another 2 hours.

(6) To detect the absorbance, the 96-well plate was placed in a chemiluminescence instrument, and the absorbance value (OD value) at 450nm was detected. The data was recorded. Cell viability = (OD value of the assay well - OD value of the blank control well)/(OD value of the solvent control well - OD value of the blank control well) × 100%.

(7) The data was imported into GraphPad PrismS for plotting. The IC50 values were calculated, and the curves were drawn.

**Cell EDU proliferation assay**

(1) Cells in the logarithmic growth phase were taken, and inoculated into a 24-well culture plate at $1 \times 10^4$ cells per well. The culture plate was cultured in a 37 °C incubator under the condition of 5% $CO_2$, and drug was added according to the assay design when the cells adhered to the wall.

(2) After the drug treatment, the Cell-Light EDU Apollo567 kit was taken out. The reagent A in the kit was diluted with 1640 complete culture medium at a ratio of 1000:1 to prepare a 50 μM EDU culture medium, 300μl of which was configured to each well of the 24-well plate. The 24-well plate was taken out from the incubator, and the original culture medium was aspirated with a vacuum aspirator. 300 μl of EDU culture medium was added to each well, and the plate was put back into the 37 °C incubator to continue culturing for 2 hours. The culture medium was discarded.

(3) About 300 μl of PBS was added to each well to wash the cells on a shaker. The cells were washed for 5 minutes each time in a total of 2 times. The purpose was to elute the EDU that was not incorporated into DNA.

(4) 150 μl of 4% paraformaldehyde fixative was added to each well, and incubated at room temperature for 30 minutes, then the fixative was discarded.

(5) 150 μl of 2 mg/ml glycine was added to each well, and incubated on a shaker for 5 minutes. Then, the glycine solution was discarded (this step was used to neutralize the excess aldehyde groups).

(6) 200 μl of PBS was added to each well to wash on a shaker for 5 minutes, and then the PBS was discarded.

(7) 150 μl of 0.5% Triton-X100 in PBS was added to each well, and incubated on a shaker for 10 minutes. PBS was added to wash once for 5 minutes.

(8) Preparation of the Apollo staining reaction solution. 140 μl of reagent B, 28 μl of reagent C, 9.3 μl of reagent D, 25 mg of reagent E, and 2400 μl of deionized water were taken and mixed thoroughly. 200 μl of the prepared Apollo staining reaction solution was added to each well, and incubated at room temperature on a shaker in dark for 30 minutes. Then, the staining reaction solution was discarded.

(9) 150 μl of 0.5% Triton-X100 was added to wash 2 to 3 times on a dehydration shaker, for 10 min each time, and the permeate was discarded. 150 μl of methanol was added to each well to wash 1 to 2 times, for 5 min each time, and the methanol was discarded. Then, PBS was added to wash once for 5 min.

(10) The reagent F was diluted with ultrapure water at a ratio of 100:1 to prepare an appropriate amount of 1×Hoechst 33342 reaction solution for staining the nuclei.

(11) 120 μl of 1×Hoechst 33342 reaction solution was added to each well, and incubated at room temperature on a shaker in dark for 30 minutes. The staining solution was discarded.

(12) 150 μl of PBS was added to each well to wash once, and the PBS was discarded. Then, 200 μl of PBS was added, and the plate was placed on an inverted fluorescence microscope to take pictures. 6 fields of view were taken for each well under a 200x field of view, and the proportion of EDU-positive cells among 100 cells was calculated. The results were imported into GraphPad Prism5 software to draw the histograms.

**Colony formation assay**

(1) The cell status was observed under a microscope. Cells in the logarithmic growth phase were selected and digested to make a cell suspension. 20 μl of the cell suspension was taken with a pipette and added to the CountStar disposable cell counting plate for cell counting, and the counting results were recorded.

(2) A 6-well culture plate was used for colony formation assay. Approximately 1,000 cells were inoculated into each

well, and the complete culture medium was added to make up the volume to 2.5 ml per well. The cells were shaked in a figure-8 pattern to disperse the cells evenly. The next day, the compound of formula (I) at different concentrations (0 μM, 50 μM, and 150 μM) were added to the corresponding wells according to the assay plan, and incubated in an incubator at 37°C with 5% $CO_2$ for about 2 weeks. The culture was terminated when visible colonies appeared.
(3) The culture plate was taken out, and the old culture medium was discarded. The cells were gently washed twice with PBS.
(4) 500 μl of 4% paraformaldehyde fixative was added to each well to fix the cells at room temperature for 15 minutes, and then the fixative was discarded.
(5) 500 μl of 0.1% crystal violet solution was added to each well, and cells were stained at room temperature for at least 15 minutes. The crystal violet was discarded, and the staining solution was slowly washed away with running water. The plate was inverted to be dried at room temperature, and pictures were taken.

**Flow cytometry for cell cycle detection**

(1) Cells were inoculated in a 6-well plate. When about 50% of the cells were adherent and fused, the compound of formula (I) at a concentration of 0 μM, 10 μM, 30 μM, and 90 μM were added, respectively. The plate was placed in an incubator at 37 °C with 5% $CO_2$ to continue culturing, and three time points were set, which were 24 h, 48 h and 72 h, respectively.
(2) After reaching the designated time point, the cell culture plate was taken out from the incubator, and placed in a biological safety cabinet. A 4ml centrifuge tube was taken and marked. The supernatant in each well was pipetted out and added to the corresponding 4ml centrifuge tube. The cells were gently washed with PBS, and the PBS was added to the corresponding centrifuge tube. An appropriate amount of trypsin was added to each well to digest the cells. When the cells were digested to a moderate degree, the old culture medium from the centrifuge tube was added to the corresponding well. The cells were gently pipetted with a pipette to form a single-cell suspension, which was pipetted back into the original centrifuge tube. The tube was immediately centrifuged at 1500rpm for 5 minutes. Then, the supernatant was discarded and the precipitate was retained.
(3) 2 ml of pre-chilled PBS was added to the centrifuge tube, and centrifuged again at 1500 rpm for 5 minutes. The supernatant was discarded, and the cell debris was removed.
(4) 1 ml of 75% ethanol was added while pipetting the cells, to make the cells appear as single as possible. Then, the cells were fixed at 4 °C overnight or stored at -20 °C for long-term storage (can be stored for 1 week).
(5) The fixed cells were taken out, and centrifuged at 1000rpm for 5 minutes. The ethanol was discarded, and pre-chilled PBS was added to wash the cells. Then, the cells were centrifuged at 1000rpm for 5min to remove the residual ethanol on the cells.
(6) A cell cycle detection kit from Wuhan Promoter Biology Co., Ltd. was used. 100 μl of RNase A was added, and incubated in a 37 °C water bath for 30 minutes.
(7) 400 μl of PI was added and mixed well, and incubated at 4 °C in dark for 30 minutes.
(8) The cell cycle was detected by upflow cytometry.

**Flow cytometry for apoptosis detection**

(1) Cells were inoculated in a 6-well plate. When the cell growth density was appropriate, four concentrations (0 μM, 10 μM, 30 μM, and 90 μM) of the compound of formula (I) were added, and placed in an incubator at 37 °C with 5% $CO_2$ to continue culturing for 72 hours.
(2) After reaching the designated time point, the cell culture plate was taken out from the incubator, and placed in a biological safety cabinet. A 4ml centrifuge tube was taken and marked. The supernatant in the 6-well plate was pipetted out and added to the corresponding 4ml centrifuge tube. The cells were gently washed with PBS, and the PBS was added to the corresponding centrifuge tube. An appropriate amount of trypsin was added to each well to digest the cells. When the cells were digested to a moderate degree, the liquid in the centrifuge tube was added to the corresponding well. The cells were gently pipetted with a pipette to disperse the cells, and was pipetted back into the original centrifuge tube. The tube was immediately centrifuged at 1500rpm for 5 minutes. Then, the supernatant was discarded and the precipitate was retained.
(3) 2ml of PBS was added to the centrifuge tube, which was centrifuged again at 1000 rpm for 5 minutes. The supernatant was discarded. This step was repeated again.
(4) The cells were suspended with 1× Binding buffer, and the cell concentration was adjusted to approximately

$1 \times 10^6$ cells/ml. 100 μl of cell suspension was added to the flow cytometry tube, and 5 μl of PI and 5 μl of Annexin V-FITC were added to each tube. The cells were gently vortexed, and incubated at room temperature in dark for 15 min.

(5) 400μl of 1×Binding buffer was further added to each tube, and the detection was performed on the flow cytometer (the detection was in need to be completed within 1 hour).

### 6. Results

#### The compound of formula (I) inhibited the proliferation of lung cancer cells

The effects of different concentrations of the compound of formula (I) (0 μM, 10 μM, 50 μM, 100 μM, and 200 μM) co-cultured with A549 cells and NCI-H460 cells for 24h, 48h and 72h on cell proliferation activity were detected by the CCK-8 method. The results showed that, the IC50 values of A549 cells for 24h, 48h and 72h are 213.2±1.22μM (Figure 1A), 55.5±1.84μM (Figure 1B), and 39.7±0.98μM (Figure 1C), respectively. The inhibition of A549 cells by the compound of formula (I) was time-concentration dependent, that is, as the concentration of the compound of formula (I) increased, the inhibitory effect increased; and as time prolonged, the inhibitory effect also increased.

The compound of formula (I) also had an inhibitory effect on the proliferation of NCI-H460 cells. The results showed that, the IC50 values of NCI-H460 cells for 24h, 48h and 72h are 125.3±1.01μM (Figure 2A), 66.4±0.87μM (Figure 2B), and 48.5±1.12 μM (Figure 2C), respectively. The inhibition of NCI-H460 cell proliferation by the compound of formula (I) was also time-concentration dependent, that is, as the concentration increased and the action time prolonged, the inhibitory effect increased.

Direct detection of DNA synthesis is one of the most accurate methods for detecting the cell proliferation. EDU can be incorporated into the newly synthesized DNA chain during the DNA replication, and a fluorescent group is labeled into the newly synthesized DNA containing EDU through a "Click" reaction, so that we can know the proliferation of cells by detecting the fluorescence. The A549 and NCI-H460 cells were treated with the compound of formula (I) at a concentration of 150 μM for 48 hours. The cell proliferation assay showed that, in the group treated with the compound of formula (I), the proportion of proliferating cells was significantly reduced compared with the CTL (the compound of formula (I) was 0 μM), and the difference was statistically significant (Figure 3, $P<0.01$). FBS stimulation may promote cell proliferation, and the compound of formula (I) can inhibit the cell proliferation effect caused by the stimulation condition of high serum concentration ($P<0.05$).

#### The compound of formula (I) inhibited the colony-forming ability of lung cancer cells

In order to clarify the effect of the compound of formula (I) on the colony-forming ability of cells, A549 and NCI-H460 cells were inoculated into a 6-well plate. Then, a medium containing the compound of formula (I) at three different concentrations (0 μM, 50 μM, and 150 μM) was co-cultured with the cells. As shown in Figure 4, compared with 0 μM, as the drug concentration increased, the colony-forming ability of the two lung cancer cells decreased significantly in a concentration-dependent manner (Figure 4).

#### The compound of formula (I) reduced the proportion of S phase of lung cancer cells

As mentioned before, the compound of formula (I) effectively inhibited the proliferation of lung cancer cells in a concentration-dependent manner. In order to further explore the reason why it inhibited the cell proliferation, we used flow cytometry technology to detect the cell cycle distribution of the lung cancer cell lines treated with the compound of formula (I). The A549 cells and NCI-H460 cells were treated with media containing different concentrations of the compound of formula (I) (0 μM, 10 μM, 30 μM, and 90 μM) for 24h, 48h, and 72h respectively. After being fixed with 75% ethanol, the cells were stained with PI, and analyzed on a flow cytometer. As shown in Figure 5, in the A549 cells, compared with the 0 μM group, the compound of formula (I) reduced the proportion of S phase of A549 cells in a dosage-dependent manner. Specifically, after 24 hours of treatment with the compound of formula (I), the proportions of S phase of cells in the 0 μM, 10 μM, 30 μM, and 90 μM groups were 31.91%, 22.94%, 12.52%, and 3.92%, respectively. After 48 hours of treatment with the compound of formula (I), the proportions of the S phase of cells in the 0 μM, 10 μM, 30 μM, and 90 μM groups were 25.09%, 24.73%, 15.96%, and 4.37%, respectively. After 72 hours of treatment with the compound of formula (I), the proportions of the S phase of cells in the 0 μM, 10 μM, 30 μM, and 90 μM groups were 20.64%, 19.77%, 10.63%, and 5.42%, respectively.

The results of NCI-H460 cells were similar to those of A549 cells. The results are shown in Figure 6: After 24 hours of treatment of NCI-H460 cells with the compound of formula (I), the proportions of S phase cells in the 0 μM, 10 μM, 30 μM, and 90 μM groups were 23.57%, 26.03%, 13.15%, and 9.76%, respectively. After 48 hours of treatment with the compound of formula (I), the proportions of S phase cells in the 0 μM, 10 μM, 30 μM, and 90 μM groups were

29.09%, 29.62%, 16.02%, and 0.97%, respectively. After 72 hours of treatment with the compound of formula (I), the proportions of S phase cells in the 0 μM, 10 μM, 30 μM, and 90 μM groups were 25.33%, 21.61%, 20.98%, and 3.36%, respectively.

**The compound of formula (I) promoted apoptosis of lung cancer cells**

We used AnnexinV-FITC and PI double staining method for apoptosis staining. FL1 channel was selected for FITC, and FL2 channel was selected for PI. Flow cytometry analysis was used to detect cell apoptosis. Q1, Q2, Q3, and Q4 represented dead cells, late apoptotic cells, early apoptotic cells, and normal cells, respectively (Figure 7). After treating A549 and NCI-H460 cells with the compound of formula (I) at different concentrations (0 μM, 10 μM, 30 μM, and 90 μM) for 72h, the flow cytometry results are as follows. Table 1 and Table 2 show the proportions of dead cells, late apoptotic cells, early apoptotic cells, and normal cells after treating the A549 and NCI-H460 cells with different concentrations of the compound of formula (I), respectively. As shown in the results in Table 1 and Table 2, when the concentration of the compound of formula (I) was low (10 μM, 30 μM), the proportions of early apoptotic cells and late apoptotic cells in A549 and NCI-H460 cells slightly increased compared with the control group, but the difference was not statistically significant. However, when the concentration of the compound of formula (I) was increased to 90 μM, the proportions of early apoptotic cells and late apoptotic cells in A549 and NCI-H460 cells both increased significantly, and the increase in the proportion of early apoptotic cells was more obvious.

**Table 1 Effects of different concentrations of the compound of formula (I) on A549 cell apoptosis**

| | 0 μM | 10 μM | 30 μM | 90 μM |
|---|---|---|---|---|
| Q1 Gated% | 0.61±0.35 | 1.07±0.51 | 1.34±0.19 | 0.31±0.14 |
| *P* | | *0.4041* | *0.1253* | *0.3811* |
| Q2 Gated% | 2.57±0.51 | 2.57±0.48 | 3.08±0.16 | 20.9±3.39 |
| *P* | | *1.000* | *0.3132* | *0.0171** |
| Q3 Gated% | 1.29±0.15 | 1.86±0.37 | 2.58±1.38 | 50.4±1.41 |
| *P* | | *0.1768* | *0.3189* | *0.0004**** |
| Q4 Gated% | 95.5±0.71 | 94.5±0.57 | 93.05±1.34 | 28.4±4.67 |
| *P* | | *0.2588* | *0.1500* | *0.0025*** |

**Table 2 Effects of different concentrations of the compound of formula (I) on NCI-H460 cell apoptosis**

| | 0 μM | 10 μM | 30 μM | 90 μM |
|---|---|---|---|---|
| Q1 Gated% | 1.98±0.93 | 1.72±0.08 | 1.75±0.45 | 0.55±0.04 |
| *P* | | *0. 7356* | *0.7868* | *0.2736* |
| Q2 Gated% | 2.66±0.95 | 3.23±2.59 | 4.79±0.37 | 9.32±0.35 |
| *P* | | *0.7975* | *0.0975* | *0.0113** |
| Q3 Gated% | 2.55±1.77 | 1.55±1.16 | 5.01±1.70 | 38.35±2.33 |
| *P* | | *0.5751* | *0.2915* | *0.0033*** |
| Q4 Gated% | 92.85±1.77 | 93.5±3.82 | 88.45±2.47 | 51.85±2.05 |
| *P* | | *0.8473* | *0.1774* | *0.0022*** |

Table 3 shows the changes in the proportion of total apoptosis after treating A549 and NCI-H460 cells with different concentrations of the compound of formula (I). It can be seen that, when the concentration of the compound of formula (I) was 90 μM, the proportion of cell apoptosis increased significantly, and this phenomenon was more obvious in A549 cells compared with NCI-H460 cells.

**Table 3 Effects of different concentrations of the compound of formula (I) on total apoptosis of A549 and NCI-H460 cells**

| Cell line | Concentration (μM)/percentage of apoptosis | | | |
|---|---|---|---|---|
| A549 | 0 μM | 10 μM | 30 μM | 90 μM |
| | 3.86±0.36 | 4.43±0.11 | 5.65±1.54 | 71.3±4.81 |

(continued)

| Cell line | Concentration (μM)/percentage of apoptosis | | | |
|---|---|---|---|---|
| NCI-H460 | 0 μM<br>5.21±2.73 | 10 μM<br>4.78±3.75 | 30 μM<br>9.8±2.06 | 90 μM<br>47.67±1.99 |

**Example 3 Assay of the inhibitory effect of the compound of formula (I) compared with cisplatin on the in vivo tumor growth of the human gastric cancer N87 transplanted tumor model in nude mice**

**Assay animals**

Female BALB/c nude mice (age: 6 to 7 weeks) were purchased from Beijing Vital River Laboratory Animal Technology Co., Ltd.. The mice were raised in an SPF animal room at a temperature of 20 to 25 °C, a relative humidity of 40% to 70%, and a cycle of 12 hours of light and 12 hours of dark. The animals had free access to water and food. After about 1 week of normal feeding, mice with good physical condition after the veterinary examination were selected for this assay. Before grouping, a marker was used to mark on the base of the animals' tail. After grouping, each animal was marked with an ear cutout.

Cisplatin (DDP) preparation

The solution was prepared before each administration. An appropriate amount of DDP was weighed and put into a glass bottle. Then, an appropriate volume of physiological saline was added to the bottle, and vortexed (may be heated in a water bath to 50 to 60 °C) to completely dissolve the drug. A solution with the DDP concentration of 0.5 $mg \cdot mL^{-1}$ was prepared.

Preparation of administration formulation of the compound of formula (I)

An appropriate amount of the compound was weighed and added to an appropriate volume of dimethylacetamide (DMA). The mixture was vortexed to prepare a stock solution (8 $mg \cdot mL^{-1}$). The stock solution was prepared once a week, divided into packages and then stored in a 2 to 8 °C refrigerator. One tube was taken out before each administration, and an appropriate volume of 30% Solutol and PEG400 was added while vortexing. The mixture was vortexed and mixed well. Then, an appropriate volume of physiological saline was added, and mixed well to give an administration formulation with a concentration of 0.4 $mg \cdot mL^{-1}$, in which the volume ratio of DMA: 30% Solutol: PEG 400: physiological saline was 5:20:20:55.

Samples of the above-mentioned administration formulation were needed to be retained, and the actual concentrations of the retained samples of the formulation were measured.

**Transplantable tumor cell lines**

Human gastric cancer cells NCI-N87 were obtained from the Cell Bank of the Type Culture Collection Committee of Chinese Academy of Sciences (cryopreserved in liquid nitrogen in the laboratory).

**NCI-N87 cell culture**

NCI-N87 cells were routinely cultured in a RPMI-1640 culture medium containing 10% fetal bovine serum under culture conditions of 37°C and 5% $CO_2$. The cells were digested with 0.25% trypsin, and passaged according to the cell growth conditions with a passaging ratio of 1:3 to 1:5.

**Animal model preparation**

The NCI-N87 cells in the logarithmic growth phase were collected, counted and resuspended in a serum-free RPMI-1640 medium, and the cell concentration was adjusted to $5 \times 10^7$ cells/mL. The cells were pipetted with a pipette to disperse them evenly, and the cells were put into a 50-mL centrifuge tube, which was placed in an ice box. A 1-mL syringe was used to draw the cell suspension, which was injected subcutaneously into the axilla of the front right limb of a nude mouse. Each animal was inoculated with 100 μL ($5 \times 10^6$ cells/mouse) to establish an NCI-N87 transplanted tumor model in nude mice. After the inoculation, the animal status and tumor growth were regularly observed, and the tumor diameter was measured using an electronic vernier caliper. The data were input into an Excel spreadsheet to

calculate the tumor volume. When the tumor volume reached 100 to 300 $mm^3$, 72 animals in good health and with similar tumor volumes were selected and divided into 9 groups (n = 8) using randomized block method. The day of grouping was regarded as the first day of the assay (D1). After the start of the assay, the tumor diameter was measured twice a week, the tumor volume was calculated, and the animal body weight was also weighed and recorded.

The calculation formula of tumor volume (TV) is as follows:

$$TV\ (mm^3) = l \times w^2/2$$

wherein *l* represents the long diameter of a tumor (mm); and *w* represents the short diameter of a tumor (mm).

**Animal grouping and drug administration**

**Table 4. Grouping and administration protocol of the drug efficacy assay in NCI-N87 transplanted tumor model**

| Group | Compound | Number of animals (n) | Dosage ($mg \cdot kg^{-1}$) | Administ ration volume ($mL \cdot kg^{-1}$) | Route of administr ation | Administr ation cycle |
|---|---|---|---|---|---|---|
| A | Blank solvent | 8 | - | 10 | i.g. | BID×28 |
| B | DDP | 8 | 4/4/5/5 | 10 | i.p. | QW×4 |
| C | Compound of formula (I) | 8 | 2 | 10 | i.g. | BID×28 |
| D | Compound of formula (I) | 8 | 6 | 10 | i.g. | BID×28 |
| E | Compound of formula (I) | 8 | 18 | 10 | i.g. | BID×28 |

**End of assay**

On the last day of the assay (D44), the animals were anesthetized using $CO_2$ inhalation 1 hour after administration (R01) and sacrificed, and tumor tissues were collected, weighed, and photographed.

**Data recording and calculation formula**

The calculation formula of tumor growth inhibition rate TGI (%) is:

$$TGI(\%) = 100\% \times [1 - (TV_{t(T)} - TV_{initial(T)})/(TV_{t(C)} - TV_{initial(C)})]$$

wherein $TV_{t(T)}$ represents the tumor volume measured each time in the treatment group; $TV_{initial(T)}$ represents the tumor volume of the treatment group when being grouped for administration; $TV_{t(C)}$ represents the tumor volume measured each time in the solvent control group; $TV_{initial(C)}$ represents the tumor volume of the solvent control group when being grouped for administration.

The calculation formula of animal weight loss rate is:

$$\text{Animal weight loss rate} = 100\% \times (BW_{initial} - BW_t)/BW_{initial}$$

wherein BWt represents the animal body weight measured each time during the administration period; $BW_{initial}$ represents the animal body weight when being grouped for administration.

**Statistical analysis method**

The assay data were calculated and processed statistically using Microsoft Office Excel 2007 software. Unless otherwise specified, the data were expressed as mean ± standard error (Mean ± SE), and t-test was used for comparison

between two groups.

**Assay observation**

During the assay, experimenters and veterinarians were needed to continuously observe the physical signs and health status of the assay animals. Any abnormal performances of the animals, such as pain, depression, reduced activity, etc., were recorded in the original assay record. If the abnormal performances of the assay animals exceeded the requirements of IACUC-related animal welfare documents, the veterinarian could determine whether to terminate the assay and notified the person in charge of the assay.

**The assay results are as follows:**

**Table 5. Tumor volume and tumor growth inhibition rate of animals in each group during the administration period**

| Group | Mean tumor volume±SE ($mm^3$) or (tumor growth inhibition rate) | | | | | | | | |
|---|---|---|---|---|---|---|---|---|---|
| | D13 | D16 | D20 | D23 | D27 | D30 | D34 | D37 | D44 |
| **A** | 138±9 | 221±22 | 302±22 | 377±26 | 526±45 | 687±43 | 902±58 | 1120±96 | 1367±118 |
| **B** | 141±9 | 194±18 (36%) | 245±22 (37%) | 276±28* (43%) | 351±22** (46%) | 413±27** (51%) | 492±33** (54%) | 580±47** (55%) | 684±45** (56%) |
| **C** | 141±9 | 180±17 (52%) | 218±17** (53%) | 253±24** (53%) | 294±30** (60%) | 332±32** (65%) | 370±41** (70%) | 450±46** (68%) | 536±52** (68%) |
| **D** | 140±8 | 183±10 (48%) | 220±11** (51%) | 255±16** (52%) | 302±24** (58%) | 348±31** (62%) | 412±37** (64%) | 508±38** (62%) | 595±57** (63%) |
| **E** | 134±8 | 175±19 (50%) | 2094±27* (54%) | 245±36* (54%) | 297±48** (58%) | 354±64** (60%) | 444±79** (59%) | 492±87** (64%) | 590±113** (63%) |

**Table 6. Body weight and weight loss rate of animals in each group during the administration period**

| Group | Mean animal body weight±SE (g) or (body weight loss rate) | | | | | | | | |
|---|---|---|---|---|---|---|---|---|---|
| | **D13** | **D16** | **D20** | **D23** | **D27** | **D30** | **D34** | **D37** | **D44** |
| **A** | 18.4±0.3 | 18.2±0.5 (1.2%) | 18.3±0.5 (0.95%) | 18.5±0.6 (-0.47%) | 19.0±0.5 (-2.8%) | 19.1±0.5 (-3.8%) | 19.3±0.5 (-4.5%) | 19.5±0.5 (-6.0%) | 19.9±0.6 (-7.9%) |
| **B** | 18.5±0.4 | 17.3±0.4 (6.2%) | 17.6±0.4 (4.8%) | 17.5±0.4 (5.1%) | 18.3±0.6 (1.1%) | 17.6±0.4 (4.7%) | 17.7±0.4 (4.3%) | 17.2±0.4 (6.8%) | 16.8±0.4 (8.9%) |
| **C** | 18.3±0.4 | 18.2±0.5 (0.41%) | 18.2±0.4 (0.27%) | 18.5±0.5 (-1.0%) | 18.7±0.5 (-2.5%) | 18.9±0.4 (-3.4%) | 19.3±0.5 (-5.8%) | 19.5±0.5 (-6.5%) | 19.2±0.6 (-5.2%) |
| **D** | 17.6±0.3 | 17.6±0.3 (0.28%) | 17.5±0.4 (0.50%) | 18.1±0.5 (-2.7%) | 18.3±0.5 (-3.7%) | 18.3±0.4 (-4.0%) | 18.5±0.4 (-5.1%) | 18.6±0.4 (-5.8%) | 19.0±0.5 (-8.0%) |
| **E** | 17.9±0.5 | 17.7±0.4 (1.0%) | 17.8±0.3 (0.56%) | 18.0±0.4 (-0.42%) | 18.1±0.3 (-1.0%) | 18.4±0.4 (-2.7%) | 18.7±0.4 (-4.3%) | 18.8±0.4 (-4.8%) | 19.1±0.4 (-6.9%) |

Note: "*" indicates that there was a significant difference in the tumor volume compared with the solvent control group ($P < 0.05$); "**" indicates that there was a highly significant difference in the tumor volume compared with the solvent control group ($P < 0.01$).

It can be seen from the above tables that, the compound of formula (I) of the present disclosure has better anti-tumor effect than that of the commonly used anti-gastric cancer drug cisplatin; the body weight of the animals in the cisplatin group decreased significantly compared with those of the blank control group, while the compound of the present disclosure had no significant effect on the body weight of the animals.

## Example 4 Quantitative study on the regulation of expression of VEGF/VEGFR/p-VEGFR

## and other proteins in multiple transplanted tumor tissues

### Assay purpose

In this assay, western blot technology was used to quantitatively study the effect of the compound of formula (I) on the signaling pathways related to tumor growth and inhibition in the digestive tract tumor NCI-N87 transplanted tumor tissues in nude mice in Example 3, aiming to explore the anti-tumor mechanism of the compound of formula (I).

### Assay materials

The tumor tissue specimens were from the animal tumor specimens in Example 3.

### Assay method

Western Blot technology was used to quantitatively detect the expression of VEGF/VEGFR, Survivin and other proteins in tumor tissues. For detailed assay methods and materials used, please refer to the reference (Molecular Cancer Research. June 2020, Volume 18, Issue 6, page 926-937) and the instructions for the Cell Signaling Technology kit. In this study, 50 μg of tumor tissue proteins were added to each well, and the working concentrations of the antibodies were diluted according to the instructions. Briefly: the tumor tissue was taken, lysed with an RIPA protein lysis solution containing 1% PMSF and mixed protease inhibitors, and centrifuged at 12000×g for 20 min to take the supernatant; the concentration of proteins was detected by BCA method; 10% SDS-PAGE gel electrophoresis was performed; the proteins from SDS-PAGE gel were transfered to a PVDF membrane by semi-dry electroporation; the membrane was blocked with a fast blocking buffer for 10 minutes, and a primary antibody at corresponding working concentration was added, and incubated at 4 °C overnight; the membrane was washed with TBST for 3 times, and incubated with a secondary antibody at room temperature for 1h; after washing the membrane three times, an image was developed and saved, and the grayscale values were analyzed with Image lab; the anti-Vinculin antibody was used as an internal reference control antibody.

### Data analysis

The grayscale of the Western blot bands were calculated with Image J software, and Graphpad Prism 5 was used for related statistical processing. A two-sided t-test was used for comparison between two groups. $P > 0.05$ means no significance (NS), $P < 0.05$ is *, $P < 0.001$ is **, and $P < 0.001$ is ***.

### Results and discussion

This assay explores the anti-tumor mechanism of the compound of formula (I) in the digestive tract tumor NCI-N87 transplanted tumor model in nude mice, which will be elaborated from three aspects including angiogenesis, cell proliferation, and apoptosis.

### Effect of the compound of formula (I) on the expression of important target proteins in the NCI-N87 transplanted tumor in nude mice and possible tumor inhibitory mechanism

In the NCI-N87 transplanted tumor model in nude mice, the anti-tumor effects of cisplatin (DDP) and the compound of formula (I) were compared. The compound of formula (I) down-regulated p-VEGFR2 (NS), down-regulated VEGFR2 ($P<0.05$) and VEGFA ($P<0.05$); down-regulated β-catenin ($P<0.05$) and CyclinD1 (NS), up-regulated PCNA (NS); up-regulated cell cycle regulatory protein P21 (NS), down-regulated the pro-apoptotic Bax ($P < 0.01$) and the anti-apoptotic protein Survivin ($P < 0.05$). DDP down-regulated p-VEGFR2 ($P < 0.05$) and VEGFA (NS), up-regulated VEGFR2 (NS), β-catenin ($P < 0.05$), and CyclinD1 (NS), down-regulated the apoptosis inhibitory protein Survivin (NS), and down-regulated cell cycle regulatory protein P21 ($P < 0.05$). Therefore, in the N87 transplanted tumor model in nude mice, the compound of formula (I) showed stronger angiogenesis inhibitory effect compared with DDP, and thus had better anti-tumor effect (Figure 8).

**Example 5: Acute toxicity assay of the orally administered test sample**

**Test sample:** the compound of formula (I).

**Preparation of the test sample**

**Preparation method and storage of solvent**

Taking the preparation of 1000 mL as an example: the 1000 mL mark was marked as the final volume on a container, and about two-thirds of the final volume of pure water at room temperature was firstly added into the container; 5 g of sodium carboxymethyl cellulose was accurately weighed, and added to the container while stirring; the volume was adjusted to the mark, and the mixture was continuelly stirred until evenly dispersed to give the desired solution.

The solution was stored airtightly at 2 °C to 8 °C for 28 days.

**Preparation method and storage of administration formulation of excipient control group**

Preparation of the administration formulation of the excipient control group: The final volume mark was marked on a container, and a required amount of excipients was weighed into the container. An appropriate amount of solvent was firstly added, stirred and mixed thoroughly, and then the solvent was further added to dilute and mix to the final volume mark. A visually uniform formulation was obtained. The formulation was stored airtightly at room temperature in dark for 24 hours, or stored airtightly at 2 °C to 8 °C in dark for 8 days.

**Preparation methods and storage of administration formulation of test sample**

The weighing and preparation of the administration formulation of the test sample were in need to be performed under yellow light.

Preparation of the administration formulation of the test sample: A mixture of the compound of formula (I) and ethanol/ethyl acetate (v/v, 1/3) with a ratio of 1:1.5 was dissolved by sonication at room temperature. 2.25 equivalents of compound of formula (I) of PVP were added, and the mixture was stirred vigorously at room temperature for 20 minutes. Then, 2 equivalents of the compound of formula (I) of lactose were added, and the mixture was stirred vigorously at room temperature for 20 minutes. The mixture was dried in an oven at 50°C and then pulverized to give a yellow powder formulation of the compound of formula (I). Then, an appropriate amount of the formulation of compound of formula (I) was weighed, and an appropriate volume of 0.5% CMC-Na aqueous solution was added. The mixture was vortexed and sonicated to mix evenly (if necessary, shear emulsification may be performed), to give an administration formulation of the test sample. The administration formulation was prepared twice a week, and 3 or 4 days of dosage was prepared each time, which was divided and stored in a 0-8 °C refrigerator for later use. When the test sample was used, a final volume mark was marked on a container, and a required amount of the test sample was weighed (weighing weight of the test sample = theoretical dosage * conversion factor) into a clean and dry container. An appropriate amount of solvent was firstly added, and the mixture was stirred and mixed thoroughly, and sonicated if necessary. The solvent was further added to the final volume mark to dilute and mix evenly, to give a visually uniform formulation. Verified by the methodology, the administration formulation of the test sample had a concentration range of 0.1 mg/mL to 60 mg/mL. The administration formulation of the test sample was stable within 24 hours when being stored airtightly at room temperature in dark, and was stable within 8 days when being stored airtightly at 2 °C to 8 °C in dark.

The administration formulations were identified by labels with different colors:

**Test animals:**

Species: ICR mice
Grade : SPF grade
Number and gender of the animals being used: 40, half male and half female;
Body weight: The body weight of the female group was from 25.9 to 29.7 g; the body weight of the male group was from 27.7 to 30.8g;
Age at the time of administration: 38 to 44 days (female), 31 to 44 days (male)
Source: Beijing Vital River Laboratory Animal Technology Co., Ltd.

License number: SCXK (Jing) 2016-0006
Quality certificate number: 110011211100519578, 110011211100519414

**Dosage setting:**

**Table 7: Dosage setting of each test sample in the toxicity assay**

| Group No. | Group | Dosage (mg/kg/d)* | Concentration (mg/mL)* | Color label | Number of animals **(F/M)** | Remark |
|---|---|---|---|---|---|---|
| 1 | Excipient control group | 12750 | 255 | White | 5/5 | **$LD_{50}$ and maximum dosage (mg/kg) were determined** |
| 2 | 300mg/kg/d dosage group | 300 | 6 | Green | 5/5 | |
| 3 | 1000mg/kg/d dosage group | 1000 | 20 | Yellow | 5/5 | |
| 4 | 3000mg/kg/d dosage group | 3000 | 60 | Red | 5/5 | |

*: represents the amount of the compound of formula (I) in group Nos. 2 to 4, and represents the amount of the excipients in group No. 1.

**Administration**

Route of administration: gavage administration

Reason for choosing the route of administration: similar to the route intended for clinical use

Administration frequency: once/day
Administration period: 1 day

The dosage was calculated based on the body weight of the animals weighed on D1 after fasting (with water access) overnight. The feed was given approximately 2 hours after drug administration.

The administration formulation in each group was stirred at room temperature for at least 10 minutes, and suspended uniformly with visual inspection before the administration. The administration formulation was required to be continually stirred during the administration.

**Observation indicators:** observation of general physical signs, body weight, food intake, animal anatomy and histopathological examination were included.

**Assay results**

There was no dead animal in the excipient control group and the 300 mg/kg/d dosage group. One male animal (No. 3205) in the 1000 mg/kg/d dosage group was found dead on D3, and no obvious abnormalities were found before death; one male animal (No. 4205) in the 3000 mg/kg/d dosage group was found to have piloerection symptoms on D2 and D3, and was found dead on D4. Compared with the body weight when being grouped, the body weight of the animal on D3 decreased significantly. After the anatomy of the above-mentioned dead animals, no obvious abnormalities were found by naked eye observation.

**Observation of general physical signs**

During the assay, no obvious abnormal symptoms were found in the clinical observation of animals in the excipient control group and the 300 mg/kg/d dosage group. One male animal was found dead in the 1000 mg/kg/d dosage group, and no obvious abnormalities were found in the dead animal. No obvious abnormal symptoms were found in the clinical observation of the other animals. One male animal was found dead in the 3000 mg/kg/d dosage group, and was found to have piloerection symptoms on D2 and D3. The other animals in this group were found to have

piloerection symptoms from D2 to D7, and return to normal on D8.

The incidence and severity of the above abnormal symptoms were found to be in a dose-response relationship, which were considered to be related to the test sample.

### Body weight

Compared with the vehicle control group in the same period, there was no significant difference in the body weight of female surviving animals in each dosage group of the test sample. The male animals in the 3000 mg/kg/d dosage group of the test sample showed a significant decrease in body weight on D3 ($P<0.05$). There was no significant difference in the body weight of the male surviving animals in each dosage group in other periods. Based on the comprehensive analysis of the clinical symptoms, this was considered to be related to the test sample.

### Food intake measurement

By analyzing the average food intake data of the animals in each dosage group, it was found that the food intake of each dosage group was basically consistent with the food intake of the vehicle control group.

### Gross anatomy examination

A gross anatomy was performed on the unplanned dead animals and the surviving animals in each group. No obvious abnormalities were found by naked eye observation, in the color, volume, texture, etc. of the organs and tissues of the animals in each group, that is, no obvious abnormal toxicity-targeted organs were found by naked eye observation. No histopathological examination was performed.

### Conclusion

Under the conditions of this assay, one male animal was dead in each of the 1000 mg/kg/d and 3000 mg/kg/d dosage groups. All animals in the 3000 mg/kg/d dosage group showed piloerection symptoms and returned to normal on Day 8, and the body weight of the male animals in this group decreased slightly on Day 3 after the administration. There were no obvious abnormalities in the food intake of the animals in each dosage group. All animals were dissected and no obvious abnormalities were found by gross naked eye observation. In summary, the maximum tolerated dose (MTD) of ICR mice after a single gavage administration of the compound of formula (I) was 300 mg/kg/d, with the $LD_{50}$>3000 mg/kg, indicating that the compound of formula (I) is very safe.

### Example 6: Toxicity dosage exploration assay of repeated gavage administration of the compound of formula (I) in ICR mice for 2 weeks

The compound of formula (I) was gavagely administered continuously to ICR mice for 14 days, to evaluate the nature, degree, and time-effect relationship of the toxic reactions that the compound of formula (I) may cause, and to provide reference information for the subsequent research.

### Test sample: Preparation of the administration formulation

The weighing and preparation of the administration formulation of the test sample were in need to be performed under yellow light.

Preparation of the administration formulation of the test sample in each dosage group: The preparation of the test sample was the same as that in Example 5. When the test sample was used, a final volume mark was marked on a container, and a required amount of the test sample was weighed (weighing weight of the test sample = theoretical dosage/conversion factor) into a container. An appropriate amount of solvent was firstly added, and the mixture was stirred and mixed thoroughly, and sonicated if necessary. The solvent was further added to the final volume mark to dilute and mix evenly, to give a visually uniform formulation, which was verified by the methodology. The assay results showed that, the administration formulation of the test sample with a concentration range of 0.1 mg/mL to 60 mg/ mL was stable within 24 hours at room temperature in dark. The administration formulation of the test sample with a concentration range of 0.01 mg/mL to 60 mg/ mL was stable within 8 days when being stored airtightly at 2 °C to 8 °C in dark.

**Table 8 The administration formulations identified by labels with different colors:**

| Group No. | Group | Dosage (mg/kg/d)* | Concentration (mg/mL)* | Color label |
|---|---|---|---|---|
| 1 | 25mg/kg/d dosage group | 25 | 1.25 | White |
| 2 | 50mg/kg/d dosage group | 50 | 2.5 | Green |
| 3 | 100mg/kg/d dosage group | 100 | 5 | Yellow |
| 4 | 300mg/kg/d dosage group | 300 | 15 | Red |
| 5 | 1000mg/kg/d dosage group | 1000 | 50 | Orange |
| *: represents the amount of the compound of formula (I). | | | | |

**Test animals:**

| | |
|---|---|
| Species | ICR mice |
| Grade | SPF grade |
| Supplier | Jinan Pengyue Experimental Animal Breeding Co., Ltd. Laboratory animal production license: SCXK (Lu) 2019 0003 |
| Number and gender | 50 females and 50 males were used for the assay, 20 animals per group |
| Expected age at the start of administration | 5 to 8 weeks |
| Expected body weight at the start of administration | Female 24-30 g, male 26-32 g |

**Assay method**

**Raising place**

After being purchased, the test animals were raised in an SPF animal room. The license number of the testing institution was SYXK (Lu) 2018 0031. The animals were raised in transparent mouse cages, with males and females separated, and with less than or equal to 5 animals in each cage.

**Raising environment and conditions**

Approximately 12 hours of light was provided per day. Dark hours could be interrupted intermittently by the demands of research-related activities. The environmental temperature and relative humidity of the animal room were monitored and recorded every day, and were controlled at 20 °C to 26 °C, and 40% to 70%, respectively.

**Feed and water**

Growth and breeding feed for SPF rats and mice were purchased from Beijing Keao Xieli Feed Co., Ltd. The animals had free access to food.
The corn cob pellet bedding was purchased from Beijing Keao Xieli Feed Co., Ltd.
Animals were free to drink the drinking water filtered and sterilized by the institution's reverse osmosis system.
The quality inspection reports of the purchased batches of feed were provide by the feed supplier. Other inspection frequencies, indicators and requirements were carried out in accordance with the center's SOP requirements.

**Quarantine and adaptation of animals**

The animals had been quarantined and adapted after arriving at the testing institution. According to the latest animal health status screening, 100 healthy animals (half male and half female) were selected for the assay after the signature and confirmation by the project leader.

**Route of administration**

Route of administration: oral gavage
Reason for choosing the route of administration: similar to the route intended for clinical use

**Administration frequency**

Administered once a day; while maintaining the same administration period every day.

**Administration cycle**

Administered continuously for 14 days

**Administration**

The dosage was calculated based on the latest animal body weight.

The administration formulation of the test sample was stirred at room temperature for at least 10 minutes, and suspended uniformly with visual inspection before the administration. The administration formulation of the test sample was required to be continually stirred during the administration.

**Observation indicators:** observation of general physical signs, detailed clinical observation, body weight, and food intake were included.

**Results:**

1. A total of 4 animals in the 1000 mg/kg/d dosage group were found dead from D3 to D6. The administration to the animals in this group was stopped and the observation was continued.
2. 13 animals were found dead in the 1000 mg/kg dosage group, 10 animals were found dead in the 300 mg/kg dosage group, and 3 animals were found dead in the 100 mg/kg dosage group. No animal was found dead in the 50 mg/kg and 25 mg/kg dosage groups.
3. No abnormal reactions were found in the surviving animals in the 100 mg/kg dosage group as well as in the animals in the 50 mg/kg and 25 mg/kg dosage groups, and their body weight increased normally.

**Example 7: Assay of inhibitory effects of the compound of formula (I) alone and in combination with the chemotherapy drug cisplatin (DDP) on tumor growth in vivo in the human gastric cancer NCI-N87 transplanted tumor model in nude mice**

The assay animals, blank solvent preparation, cisplatin (DDP) preparation, preparation of the administration formulation of the compound of formula (I), transplantable tumor cell lines, NCI-N87 cell culture, animal model preparation, assay procedures and statistical analysis methods were the same as those in Example 3.

**Animal grouping and drug administration**

**Table 9. Grouping and administration protocol of the drug efficacy assay in NCI-N87 transplanted tumor model**

| Group | Sample | Dosage (mg·kg$^{-1}$) | Administration volume (mL·kg$^{-1}$) | Route of administration | Administration cycle |
|---|---|---|---|---|---|
| 1 | Blank solvent | - | 10 | PO | QD × 32 |
| 2 | DDP | 4/2/1/4 | 10 | IP | 4 mg·kg-1 (D13, D20) / 2 mg·kg-1 (D27) / 1 mg·kg-1 (D34) / 4 mg·kg-1 (D41) |
| 3 | R01 | 16 | 10 | PO | QD × 32 |
| 4 | R01 + DDP | R01: 3 | 10 | PO | QD × 32 |
| | | DDP: 4/2/1/4 | 10 | IP | 4 mg·kg-1 (D13, D20) / 2 mg·kg-1 (D27) / 1 mg·kg-1 (D34) / 4 mg·kg-1 (D41) |
| 5 | R01 + DDP | R01: 6 | 10 | PO | QD × 32 |
| | | DDP: 4/2/1/4 | 10 | IP | 4 mg·kg-1 (D13, D20) / 2 mg·kg-1 (D27) / 1 mg·kg-1 (D34) / 4 mg·kg-1 (D41) |
| 6 | R01 + DDP | R01: 16 | 10 | PO | QD × 32 |
| | | DDP: 4/2/1/4 | 10 | IP | 4 mg·kg-1 (D13, D20) / 2mg·kg-1 (D27) / 1mg·kg-1 (D34) / 4 mg·kg-1 (D41) |
| 7 | R01 + DDP | R01: 8 | 10 | PO | BID × 28.5 (D12 afternoon to D19 morning, D22 afternoon to D43 |
| | | DDP: 5 | 10 | IP | D13, D16 |

The assay results are as follows:

## Table 10. Tumor volume and tumor growth inhibition rate of animals in each group during the administration period

| Group | Mean tumor volume±SE (mm$^3$) or tumor growth inhibition rate (TGI) | | | | | | | | | |
|---|---|---|---|---|---|---|---|---|---|---|
| | D12 | D16 | D19 | D23 | D26 | D30 | D33 | D37 | D40 | D44 |
| Solvent control group | 155±5 | 282±23 | 359±24 | 477±37 | 632±52 | 900±76 | 1047±100 | 1262±131 | 1423±150 | 1598±162 |
| DDP | 155±6 | 241±6<br>15% | 294±6*<br>18% | 341±13**<br>28% | 419±22**<br>34% | 543±32**<br>40% | 642±35**<br>39% | 810±61*<br>36% | 911±80*<br>36% | 1035±95*<br>35% |
| R01 | 155±4 | 200±6**<br>29% | 263±14**<br>27% | 336±18**<br>30% | 421±26**<br>33% | 509±46**<br>43% | 577±52**<br>45% | 681±63**<br>46% | 758±74**<br>47% | 871±94**<br>45% |
| R01 (3 mg/kg) + DDP | 156±4 | 220±6*#<br>22% | 282±13*<br>21% | 346±21*<br>27% | 418±26**<br>34% | 489±35**<br>46% | 554±41**<br>47% | 664±50**<br>47% | 744±48**<br>48% | 804±46**<br>50% |
| R01 (6 mg/kg) + DDP | 153±6 | 212±8*#<br>25% | 270±13**<br>25% | 324±16**<br>32% | 405±22**<br>36% | 463±26**<br>49% | 511±35**#<br>51% | 571±59**#<br>55% | 644±66**#<br>55% | 692±73**#<br>57% |
| R01 (16 mg/kg) + DDP | 150±4 | 230±8<br>18% | 299±12<br>17% | 353±25*<br>26% | 444±21**<br>30% | 535±26**<br>41% | 593±30**<br>43% | 641±32**#<br>49% | 706±52**#<br>50% | 768±69**#<br>52% |
| R01 (BID) + DDP | 153±6 | 197±12**##<br>30% | 249±18**#<br>31% | 286±23**<br>40% | 326±33**#<br>48% | 409±30**#<br>55% | 454±31**##<br>57% | 508±31**##<br>60% | 581±27**##<br>59% | 619±45**##<br>61% |

Note: "*" indicates that there was a significant difference in the tumor volume compared with the solvent control group ($P < 0.05$); "**" indicates that there was a highly significant difference in the tumor volume compared with the solvent control group ($P < 0.01$).

## Table 11. Body weight and body weight loss rate of animals in each group during the administration period

| Group | Animal body weight±SE (g) or body weight loss rate (%) | | | | | | | | | |
|---|---|---|---|---|---|---|---|---|---|---|
| | D12 | D16 | D19 | D23 | D26 | D30 | D33 | D37 | D40 | D44 |
| Solvent control group | 17.7±0.5 | 18.3±0.4 | 18.4±0.2 | 18.5±0.4 | 18.2±0.3 | 18.8±0.3 | 19.0±0.4 | 18.6±0.3 | 18.8±0.3 | 19.0±0.2 |
| | | -3.6% | -3.9% | -4.3% | -2.8% | -6.0% | -7.2% | -5.1% | -6.0% | -7.1% |
| DDP | 18.6±0.5 | 17.9±0.3 | 18.0±0.4 | 16.9±0.5 | 17.9±0.5 | 17.5±0.3 | 18.6±0.4 | 18.9±0.4 | 19.2±0.4 | 18.3±0.3 |
| | | 3.9% | 3.3% | 9.2% | 3.4% | 6.0% | 0.090% | -2.0% | -3.1% | 1.4% |
| R01 | 18.6±0.3 | 19.2±0.3 | 18.9±0.5 | 18.9±0.7 | 18.7±0.5 | 18.9±0.4 | 19.1±0.5 | 19.2±0.5 | 19.2±0.4 | 19.4±0.5 |
| | | -3.4% | -1.9% | -1.7% | -0.54% | -2.0% | -2.7% | -3.7% | -3.7% | -4.3% |
| R01 (3 mg/kg) + DDP | 18.1±0.5 | 17.5±0.4 | 17.2±0.5 | 16.3±0.4 | 16.1±0.6 | 16.7±0.5 | 17.4±0.6 | 17.1±0.6 | 17.9±0.5 | 16.0±0.6 |
| | | 3.5% | 5.3% | 10% | 11% | 7.7% | 4.3% | 5.3% | 1.6% | 12% |
| R01 (6 mg/kg) + DDP | 18.2±0.4 | 18.4±0.3 | 17.7±0.3 | 16.5±0.2 | 17.5±0.4 | 17.2±0.2 | 17.2±0.7 | 17.7±0.5 | 17.2±0.8 | 16.3±0.6 |
| | | -1.1% | 2.7% | 9.4% | 4.1% | 5.9% | 5.5% | 2.7% | 5.5% | 11% |
| R01 (16 mg/kg) + DDP | 17.9±0.4 | 17.6±0.3 | 17.5±0.4 | 16.2±0.6 | 17.0±0.8 | 17.3±0.7 | 17.7±0.8 | 17.0±0.6 | 17.8±0.6 | 16.8±0.5 |
| | | 1.9% | 2.2% | 10% | 5.1% | 3.5% | 0.93% | 5.1% | 0.84% | 6.2% |
| R01 (BID) + DDP | 18.2±0.5 | 17.7±0.3 | 15.0±0.6 | 14.9±0.5 | 15.2±0.9 | 17.1±0.7 | 18.0±0.7 | 17.9±1.0 | 17.6±1.3 | 17.5±0.9 |
| | | 2.4% | 17% | 18% | 16% | 5.9% | 94% | 1.4% | 3.3% | 3.8% |

## Table 12. Serum UREA content of animals in each group (mmol serum$^{-1}$)

| Group \ Animal No. | No.1 | No.2 | No.3 | No.4 | No.5 | No.6 | Mean | SE | P (vs. solvent control group) | P (vs. group administrated with DDP alone) |
|---|---|---|---|---|---|---|---|---|---|---|
| Solvent control group | 7.31 | 7.90 | 8.25 | 9.48 | 7.59 | 8.74 | 8.21 | 0.33 | / | / |
| DDP | 8.06 | 12.81 | 15.45 | 8.28 | 10.47 | 6.62 | 10.28 | 1.36 | 1.70E-01 | / |
| R01 | 6.44 | 7.73 | 10.36 | 6.65 | 9.42 | 6.36 | 7.83 | 0.69 | 6.26E-01 | 1.39E-01 |
| R01 (3 mg/kg) + DDP | 7.31 | 6.30 | 7.35 | 6.99 | 8.71 | 6.56 | 7.20 | 0.35 | 5.97E-02 | 5.30E-02 |
| R01 (6 mg/kg) + DDP | 5.79 | 8.86 | 6.40 | 5.37 | 7.32 | 6.28 | 6.67 | 0.51 | 2.96E-02 | 3.23E-02 |
| R01 (16 mg/kg) + DDP | 4.94 | 6.94 | 4.93 | 6.83 | 5.40 | 5.08 | 5.69 | 0.39 | 5.36E-04 | 8.71E-03 |
| R01 (BID) + DDP | — | 6.31 | 6.65 | 8.13 | 6.90 | 4.94 | 6.59 | 0.51 | 2.18E-02 | 4.34E-02 |

It can be seen from the above data that, R01 alone had a significant inhibitory effect on the growth of the NCI-N87 transplanted tumor in nude mice under the conditions of this assay, and its anti-tumor effect was greater than that of the group administrated with DDP alone. R01 at dosages of 3, 6, and 16 mg/kg (QD) were used in combination with DDP, respectively. All of the combinations had better efficacy than that of DDP alone, and reduced the serum UREA level in mice. The serum UREA level of animals in the combination groups had a negative correlation with the dosage of R01, suggesting that R01 may have a certain protective and preventive effect on the kidney damage caused by DDP. In addition, the efficacy of the R01 (8 mg/kg, BID) + DDP group was slightly better than or equivalent to that of the other administration groups.

**Example 8: Assay of synergistic inhibitory effect of the compound of formula (I) and DDP on tumor growth in vivo in human esophageal cancer ECA109 transplanted tumor model in nude mice**

The assay animals, calculation of tumor volume (TV), end of assay, data recording, calculation formula of tumor growth inhibition rate TGI (%), and statistical analysis method were the same as those in Example 3; the preparation of the administration formulation of the compound of formula (I) was the same as that in Example 5.

Blank solvent preparation

An appropriate amount of CMC-Na solid was weighed, and an appropriate volume of deionized water was firstly added. The mixture was vortexed and stirred until the solid was completely dispersed and evenly distributed, to give a 0.5% CMC-Na aqueous solution, which was stored at 2 to 8 °C in a refrigerator.

Preparation of administration test solution of cisplatin (DDP)

An appropriate amount of DDP was weighed and put into a glass bottle, and an appropriate amount of physiological saline was added to the bottle. The mixture was vortexed and sonicated to completely dissolve the drug, and

a DDP solution with a concentration of 0.5 mg/mL was prepared in the bottle. This solution was prepared before each administration.

Transplantable tumor cell lines

Human esophageal cancer cells ECA109 were obtained from the Cell Bank of Wuhan University (CCTCC, cryopreserved in liquid nitrogen in the laboratory).

Cell culture

ECA109 cells were routinely cultured in a RPMI-1640 culture medium containing 10% fetal bovine serum under culture conditions of 37°C and 5% $CO_2$. The cells were digested with 0.25% trypsin, and passaged according to the cell growth conditions with a passaging ratio of 1:3 to 1:6.

Animal model preparation and tumor volume determination

The ECA109 cells in the logarithmic growth phase were collected, counted and resuspended in 50% of serum-free RPMI-1640 medium and 50% of Matrigel, and the cell concentration was adjusted to $0.5\times10^8$ cells/mL. The cells were pipetted with a pipette to disperse them evenly, and the cells were put into a 50-mL centrifuge tube, which was placed in an ice box. A 1-mL syringe was used to draw the cell suspension, which was injected subcutaneously into the axilla of the front right limb of a NOD/SCID mouse. Each animal was inoculated with 200 μL ($1.0\times10^7$ cells/mouse) to establish an ECA109 transplanted tumor model in nude mice. After the inoculation, the animal status and tumor growth were regularly observed, and the tumor diameter was measured using an electronic vernier caliper. The data were input into an Excel spreadsheet to calculate the tumor volume. When the tumor volume reached 100 to 300 $mm^3$, animals in good health and with similar tumor volumes were selected and divided into groups using randomized block method. The day of grouping was regarded as the first day of the assay (D1). After the start of the assay, the tumor diameter was measured twice a week, the tumor volume was calculated, and the animal body weight was also weighed and recorded.

Animal grouping and drug administration

**Table 13. Grouping and administration protocol of the drug efficacy assay in ECA109 transplanted tumor model**

| Group | Compound | Number of animals (n) | Dosage ($mg\cdot kg^{-1}$) | Administration volume ($mL\cdot kg^{-1}$) | Route of administration | Administration cycle |
|---|---|---|---|---|---|---|
| A | Solvent control | 8 | - | 10 | i.g. | BID×34.5 |
| B | DDP | 8 | 5 | 10 | i.p. | QW×4 |
| C | R01 formulation | 8 | 18 | 10 | i.g | BID×35 |
| D | R01 formulation +DDP | 8 | R01: 18 | 10 | i.g | BID×35 |
| | | | DDP: 5 | 10 | i.p. | QW×4 |

**The assay results are as follows:**

**Table 14. Tumor volume and tumor growth inhibition rate of animals in each group during the administration period**

| Group | Mean tumor volume±SE ($mm^3$) and (tumor growth inhibition rate (TGI)) | | | | | | | | | | |
|---|---|---|---|---|---|---|---|---|---|---|---|
| | D1 | D5 | D8 | D12 | D15 | D19 | D22 | D26 | D29 | D33 | D36 |
| A | 193±9 | 225±12 | 273±11 | 301±9 | 342±7 | 358±7 | 374±7 | 392±8 | 421±11 | 439±12 | 445±18 |
| B | 193±9 | 204±5 (64%) | 203±11** (87%) | 215±10** (79%) | 224±8** (79%) | 247±8** (67%) | 259±8** (63%) | 276±9** (58%) | 285±9** (59%) | 259±12** (73%) | 292±11** (60%) |
| C | 193±10 | 195±6* (93%) | 207±17** (82%) | 207±17** (87%) | 204±18** (92%) | 192±18** (101%) | 185±14** (104%) | 181±14** (106%) | 170±14** (110%) | 154±11** (116%) | 185±12** (103%) |
| D | 197±11 | 171±6**## (184%) | 172±7**# (132%) | 161±11**## (134%) | 161±8**## (124%) | 150±9**## (129%) | 147±11**## (128%) | 154±11**## (122%) | 155±11**## (119%) | 143±7**## (122%) | 161±10**## (114%) |

Note: "*" indicates that there was a significant difference in the tumor volume compared with the solvent control group ($P < 0.05$); "**" indicates that there was a highly significant difference in the tumor volume compared with the solvent control group ($P < 0.01$).

**Table 15. Body weight and body weight loss rate of animals in each group during the administration period**

| Group | Mean animal body weight±SE (g) and body weight loss rate (%) | | | | | | | | | | |
|---|---|---|---|---|---|---|---|---|---|---|---|
| | D1 | D5 | D8 | D12 | D15 | D19 | D22 | D26 | D29 | D33 | D36 |
| A | 20.3±0.5 | 21.3±0.4 (-4.7%) | 20.9±0.4 (-2.6%) | 20.9±04 (-2.8%) | 22.2±0.3 (-9.0%) | 22.8±0.5 (-12%) | 22.5±0.3 (-11%) | 22.6±0.3 (-11%) | 22.4±0.3 (-10%) | 22.6±0.3 (-11%) | 22.8±0.2 (-12%) |
| B | 20.8±0.4 | 20.3±0.6 (2.1%) | 20.5±0.4 (1.4%) | 19.4±0.4 (6.7%) | 19.9±0.3 (4.3%) | 19.0±0.3 (8.5%) | 19.5±0.4 (5.9%) | 17.7±0.3 (15%) | 17.9±0.3 (14%) | 18.7±0.4 (10%) | 18.7±0.3 (10%) |
| C | 20.8±0.3 | 20.6±0.3 (0.60%) | 20.2±0.3 (2.6%) | 20.4±0.3 (1.7%) | 20.3±0.3 (2.3%) | 21.0±0.3 (-1.1%) | 20.9±0.4 (-0.78%) | 20.8±0.2 (-0.30%) | 21.0±0.4 (-1.3%) | 21.1±0.3 (-1.6%) | 20.9±0.3 (-0.54%) |
| D | 20.5±0.5 | 19.8±0.3 (3.7%) | 19.2±0.3 (6.3%) | 17.8±0.4 (13%) | 18.2±0.4 (11%) | 17.4±0.4 (15%) | 18.1±0.5 (12%) | 17.0±0.5 (17%) | 17.0±0.5 (17%) | 17.7±0.4 (14%) | 17.9±0.4 (13%) |

**Table 16. Serum UREA levels of animals in each group at the end of the assay**

| Group | Dosage ($mg \cdot kg^{-1}$) | Number of animals at the beginning/at the end | Serum UREA (mmol/L) Mean ± SE | P(vs. A) | P (vs. B) | P (vs. C) |
|---|---|---|---|---|---|---|
| A | - | 8/8 | 10.00±0.32 | — | — | — |
| B | 5 | 8/8 | 13.43±1.04 | 7.13E-03 | — | — |
| C | 18 | 8/8 | 9.12±0.31 | 6.90E-02 | 1.41E-03 | 4.89E-01 |
| D | R01: 18<br>DDP: 5 | 8/8 | 12.43±0.62 | 3.59E-03 | 4.02E-01 | — |

It can be seen from the above data that, R01 formulation alone had a significant inhibitory effect on the growth of the ECA109 transplanted tumor in nude mice under the conditions of this assay, and its anti-tumor effect was greater than that of the group administrated with DDP alone. R01 formulation with a dosage of 18 mg/kg was combined with DDP, which had better efficacy than that of DDP alone or R01 alone. The body weight loss rate of the animals in the combination group was similar to that in the group administrated with DDP alone, indicating that the combination of R01 and DDP does not lead to an increase in the toxicity of DDP. The serum urea test results after the last administration showed that, the urea level in the R01 formulation + DDP group was lower than that of the group administrated with DDP alone, indicating that the combination of R01 formulation and DDP had a certain protective or alleviating effect on the kidney damage caused by DDP administration.

**Example 9: Assay of synergistic inhibitory effect of the compound of formula (I) and cisplatin on tumor growth in vivo in human non-small cell lung cancer A549 transplanted tumor model in nude mice**

The assay animals (except that the normal feeding time was 10 days), preparation of blank solvent, preparation of cisplatin (three concentrations were prepared: 0.4, 0.5 or 0.7 mg/mL), calculation of tumor volume (TV), end of assay, data recording, calculation formula of tumor growth inhibition rate TGI (%), statistical analysis method, and assay observation were the same as those in Example 3; the preparation of the administration formulation of the compound of formula (I) was the same as that in Example 5.

Cell lines

Human non-small cell lung cancer cells A549 were obtained from the Cell Bank of the Type Culture Collection Committee of Chinese Academy of Sciences (CAS, cryopreserved in liquid nitrogen in this assay).

Cell culture

A549 cells were routinely cultured in a F12K culture medium containing 10% fetal bovine serum under culture conditions of 37°C and 5% $CO_2$. The cells were digested with 0.25% trypsin, and passaged 2 to 3 times a week according to the cell growth conditions with a passaging ratio of 1:3 to 1:5.

Animal model preparation and tumor volume determination

On D1 of the assay, the A549 cells in the logarithmic growth phase were collected, counted and resuspended in PBS, and the cell concentration was adjusted to $8\times10^7$ cells/mL. The cells were pipetted with a pipette to disperse them evenly, and the cells were put into a 50-mL centrifuge tube, which was placed in an ice box. A 1-mL syringe was used to draw the cell suspension, which was injected subcutaneously into the axilla of the front right limb of a nude mouse. Each animal was inoculated with 100 μL ($8\times10^6$ cells/mouse) to establish an A549 transplanted tumor model in nude mice. After the inoculation, the animal status and tumor growth were regularly observed. On D18, the tumor diameter was measured using an electronic vernier caliper. The data were input into an Excel spreadsheet to calculate the tumor volume. Tumor-bearing mice in good health and with similar tumor volumes (101 to 156 $mm^3$) were selected and divided into groups using randomized block method. After the start of the assay, the tumor diameter was measured regularly, the tumor volume was calculated, and the animal body weight was also weighed and recorded.

Sample collection

On the last day of the assay, $CO_2$ inhalation anesthesia was used on the animals 1 hour after the administration (R01). Blood was collected through the heart, and a part of the whole blood was added with EDTA-K2 for anticoagulation. The plasma was separated by centrifugation at 4 °C and 1500g for 10 minutes. The plasma was collected, and stored in a -40 °C to -20 °C freezer (if necessary, it can be used for the determination of the plasma drug concentration). No anticoagulant was added to the other part of the whole blood, and the serum was separated by centrifugation after the blood coagulated, which was used for the detection of blood urea nitrogen. After the blood collection, the tumor tissue was collected, weighed and photographed. Then, the tumor was divided into two parts, quickly cryopreserved by liquid nitrogen, and transferred to a -90 °C to -60 °C freezer for storage. Bilateral kidneys were collected and fixed in 10% formalin.

Animal grouping and drug administration

**Table 17. Grouping and administration protocol of the drug efficacy assay in A549 transplanted tumor model**

| Group | Compound | Number of animals (n) | Dosage ($mg\cdot kg^{-1}$) | Administration volume ($mL\cdot kg^{-1}$) | Route of administration | Administration cycle |
|---|---|---|---|---|---|---|
| A | Solvent control | 8 | - | 10 | i.g. | BID×28 |
| B | DDP | 8 | 4 | 10 | i.p. | QW×4 |
| C | DDP | 8 | 5/7/7/7 | 10 | i.p. | QW×4 |
| D | R01 formulation | 8 | 18 | 10 | i.g | BID×28 |
| E | R01 formulation | 8 | 36 | 10 | i.g. | BID×28 |
| F | R01 formulation +DDP | 8 | R01: 18<br>DDP: 5/7/7/7 | 10<br>10 | i.g<br>i.p. | BID×28<br>QW×4 |
| G | R01 formulation +DDP | 8 | R01: 36<br>DDP: 4 | 10<br>10 | i.g<br>i.p. | BID×28<br>QW×4 |

**The assay results are as follows:**

## Table 18. Tumor volume and tumor growth inhibition rate of animals in each group during the administration period

| Group | Mean tumor volume±SE ($mm^3$) and (tumor growth inhibition rate (TGI)) | | | | | | | | |
|---|---|---|---|---|---|---|---|---|---|
| | D18 | D21 | D25 | D28 | D32 | D35 | D39 | D42 | D46 |
| A | 128±5 | 210±5 | 397±13 | 540±15 | 725±17 | 858±28 | 1259±48 | 1400±74 | 1641±87 |
| B | 127±6 | 210±10 (-1.0%) | 297±20** (37%) | 418±28** (29%) | 561±35** (27%) | 670±56** (26%) | 914±89** (30%) | 1110±119 (23%) | 1333±176 (20%) |
| C | 130±5 | 208±11 (4.7%) | 308±23** (34%) | 428±30** (27%) | 562±41** (28%) | 696±52* (22%) | 892±86** (33%) | 1099±85* (24%) | 1236±32* (27%) |
| D | 127±5 | 185±8* (30%) | 291±19** (39%) | 379±24** (39%) | 485±26** (40%) | 585±49** (37%) | 701±64** (49%) | 786±84** (48%) | 952±108** (46%) |
| E | 129±5 | 182±10* (37%) | 270±20** (48%) | 379±30** (39%) | 485±33** (40%) | 573±42** (39%) | 698±54** (50%) | 786±49** (48%) | 914±61** (48%) |
| F | 129±6 | 179±11* (39%) | 266±21** (49%) | 352±17** (46%) | 454±35** (46%) | 495±42** (50%) | 581±57** (60%) | 714±71** (54%) | 766±80** (58%) |
| G | 128±5 | 179±7** (38%) | 257±16** (52%) | 349±21** (46%) | 424±30** (50%) | 493±40** (50%) | 681±50** (51%) | 756±54** (51%) | 950±92** (46%) |

Note: "*" indicates that there was a significant difference in the tumor volume compared with the solvent control group ($P < 0.05$); "**" indicates that there was a highly significant difference in the tumor volume compared with the solvent control group ($P < 0.01$).

## Table 19. Body weight and body weight loss rate of animals in each group during the administration period

| Group | Mean animal body weight±SE (g) and body weight loss rate (%) | | | | | | | | |
|---|---|---|---|---|---|---|---|---|---|
| | D18 | D21 | D25 | D28 | D32 | D35 | D39 | D42 | D46 |
| A | 18.5±0.5 | 18.0±0.5 (2.7%) | 18.6±0.5 (-0.41%) | 19.0±0.5 (-2.4%) | 19.5±0.6 (-5.3%) | 19.8±0.5 (-6.9%) | 20.2±0.4 (-9.0%) | 20.9±0.5 (-13%) | 21.1±0.4 (-14%) |
| B | 18.4±0.4 | 18.3±0.5 (0.81%) | 18.1±0.3 (1.7%) | 18.5±0.3 (-0.14%) | 18.7±0.3 (-1.4%) | 19.3±0.3 (-4.9%) | 19.3±0.3 (-4.6%) | 19.8±0.5 (-7.7%) | 19.5±0.5 (-5.6%) |
| C | 18.5±0.3 | 18.4±0.3 (0.61%) | 18.5±0.3 (0.00%) | 18.9±0.3 (-2.4%) | 18.3±0.4 (1.3%) | 19.2±0.4 (-4.0%) | 18.0±0.3 (2.5%) | 18.8±0.4 (-1.5%) | 17.1±0.5 (7.4%) |
| D | 18.4±0.6 | 18.1±0.5 (1.5%) | 18.6±0.6 (-1.1%) | 18.6±0.6 (-1.0%) | 18.7±0.5 (-2.0%) | 18.9±0.6 (-2.9%) | 19.3±0.5 (-4.9%) | 19.7±0.6 (-7.1%) | 20.1±0.6 (-9.4%) |
| E | 18.4±0.2 | 18.2±0.2 (1.0%) | 18.6±0.2 (-1.1%) | 18.5±0.2 (-0.82%) | 18.7±0.3 (-1.8%) | 18.7±0.3 (-1.8%) | 19.3±0.3 (-5.2%) | 19.8±0.3 (-7.9%) | 20.1±0.4 (-9.5%) |
| F | 17.9±0.2 | 17.9±0.1 (0.28%) | 17.7±0.2 (1.0%) | 18.2±0.2 (-1.7%) | 17.3±0.3 (3.4%) | 17.8±0.4 (0.84%) | 16.8±0.5 (6.3%) | 17.2±0.5 (4.3%) | 16.1±0.5 (10%) |
| G | 18.2±0.4 | 18.1±0.4 (0.55%) | 18.3±0.4 (-0.62%) | 18.6±0.4 (-2.3%) | 18.6±0.4 (-2.3%) | 18.9±0.4 (-4.3%) | 18.3±0.4 (-0.48%) | 19.0±0.4 (-4.6%) | 18.3±0.4 (-0.69%) |

**Table 20. Serum urea nitrogen (BUN) levels of animals in each group at the end of the assay**

| Group | Dosage ($mg \cdot kg^{-1}$) | Administration method | Number of animals at the beginning/end | BUN (mmol/L) Mean ± SE | P(vs. A) | P (vs. B/C) |
|---|---|---|---|---|---|---|
| A | - | i.g., BID×28 | 8/8 | 21.87±0.85 | — | — |
| B | 4 | i.p., QW×4 | 8/8 | 21.17±1.43 | 6.81E-01 | — |
| C | 5/7/7/7 | i.p., QW×4 | 8/8 | 28.56±1.79 | 4.55E-03 | — |
| D | 18 | i.g., BID×28 | 8/8 | 22.51±1.24 | 6.78E-01 | — |
| E | 36 | i.g., BID×28 | 8/8 | 22.69±1.17 | 5.79E-01 | — |
| F | R01: 18<br>DDP: 5/7/7/7 | i.g., BID×28<br>i.p., QW×4 | 8/8 | 19.02±2.40 | 2.81E-01 | 6.57E-03 |
| G | R01: 36<br>DDP: 4 | i.g., BID×28<br>i.p., QW×4 | 8/8 | 20.05±0.88 | 1.60E-01 | 5.15E-01 |

It can be seen from the above data that DDP at dosages of 4 $mg \cdot kg^{-1}$ (QWmg) and 5/7/7/7 $mg \cdot kg^{-1}$ (QW) had no significant effect on the growth of the A549 transplanted tumor in nude mice. The R01 formulation alone at dosages of 18 $mg \cdot kg^{-1}$ BID and 36 $mg \cdot kg^{-1}$ BID had a significant inhibitory effect on the growth of the A549 transplanted tumor in nude mice. The combination groups of R01 formulation (18 $mg \cdot kg^{-1}$ BID) + DDP (5/7/7/7 $mg \cdot kg^{-1}$ QW) and R01 formulation (36 $mg \cdot kg^{-1}$ BID) + DDP (4 $mg \cdot kg^{-1}$ QW) had a significant inhibitory effect on the growth of A549 transplanted tumor in nude mice. There was a significant difference ($P < 0.05$) or a highly significant difference ($P < 0.01$) in the relative tumor volume or tumor weight between the two combination groups and the group administrated with the corresponding dosage of DDP alone. The body weight loss rate of animals in each combination group was similar to that of the group administrated with the corresponding dosage of DDP alone, indicating that the combination of R01 and DDP does not lead to an increase in DDP toxicity, and R01 may have a certain protective effect on the kidney damage caused by DDP.

**Example 10: Assay of synergistic inhibitory effect of the compound of formula (I) and cisplatin on tumor growth in vivo in human colorectal cancer HT29 transplanted tumor model in nude mice**

The assay animals, preparation of blank solvent, preparation of cisplatin (DDP), calculation of tumor volume (TV), end of assay, data recording, calculation formula of tumor growth inhibition rate TGI (%), statistical analysis method, and assay observation were the same as those in Example 3; the preparation of the administration formulation of the compound of formula (I) was the same as that in Example 3.

**Cell lines**

The human colorectal cancer cell lines HT-29 were obtained from the Cell Bank of the Type Culture Collection Committee of Chinese Academy of Sciences (CAS, cryopreserved in liquid nitrogen in this assay).

**Cell culture**

HT-29 cells were routinely cultured in a McCoy cryopreservation medium containing 10% fetal bovine serum under culture conditions of 37°C and 5% $CO_2$. The cells were digested with 0.25% trypsin, and passaged according to the cell growth conditions with a passaging ratio of 1:3 to 1:4.

**Animal model preparation and tumor volume determination**

On D1 of the assay, the HT-29 cells in the logarithmic growth phase were collected, counted and resuspended in serum-free McCoy, and the cell concentration was adjusted to $4\times10^7$ cells/mL. The cells were pipetted with a pipette to disperse them evenly, and the cells were put into a 50-mL centrifuge tube, which was placed in an ice box. A 1-mL syringe was used to draw the cell suspension, which was injected subcutaneously into the axilla of the front right limb of a nude mouse. Each animal was inoculated with 100 $\mu$L ($4\times10^6$ cells/mouse) to establish a HT-29 transplanted tumor model in nude mice. After the inoculation, the animal status and tumor growth were regularly observed. On D12, the tumor diameter was measured using an electronic vernier caliper. The data were input into an Excel spreadsheet to calculate the tumor volume. Tumor-bearing mice in good health and with similar tumor volumes (103 to 179 $mm^3$) were

selected and divided into groups using randomized block method. After the start of the assay, the tumor diameter was measured regularly, the tumor volume was calculated, and the animal body weight was also weighed and recorded.

**Animal grouping and drug administration**

**Table 21. Grouping and administration protocol of the drug efficacy assay in HT-29 transplanted tumor model**

| Group | Compound | Number of animals (n) | Dosage ($mg \cdot kg^{-1}$) | Administration volume ($mL \cdot kg^{-1}$) | Route of administration | Administration cycle |
|---|---|---|---|---|---|---|
| A | Blank solvent | 8 | - | 10 | i.g. | BID×28 |
| B | DDP | 8 | 5 | 10 | i.p. | QW×3 |
| C | R01 | 8 | 18 | 10 | i.g. | BID×28 |
| D | R01+DDP | 8 | R01：18<br>DDP：5 | 10<br>10 | i.g.<br>i.p. | BID×28<br>QW×3 |

**The assay results are as follows:**

**Table 22. Tumor volume and tumor growth inhibition rate of animals in each group during the administration period**

| Group | Mean tumor volume±SE ($mm^3$) or (tumor growth inhibition rate) | | | | | | | | |
|---|---|---|---|---|---|---|---|---|---|
| | D12 | D15 | D19 | D22 | D26 | D29 | D33 | D36 | D40 |
| A | 132±8 | 206±17 | 386±18 | 524±25 | 710±26 | 932±38 | 1223±58 | 1354±79 | 1523±93 |
| B | 131±8 | 178±10<br>(37%) | 263±11**<br>(48%) | 383±25**<br>(36%) | 484±28**<br>(39%) | 582±25**<br>(44%) | 693±34**<br>(48%) | 777±38**<br>(47%) | 896±56**<br>(45%) |
| C | 134±9 | 178±7<br>(40%) | 280±9**<br>(42%) | 393±19**<br>(34%) | 532±31**<br>(31%) | 631±40**<br>(38%) | 832±59**<br>(36%) | 903±73**<br>(37%) | 1070±94**<br>(33%) |
| D | 132±8 | 163±16<br>(57%) | 241±26**<br>(57%) | 321±28**<br>(52%) | 403±35**<br>(53%) | 475±39**<br>(57%) | 555±55**<br>(61%) | 611±57**<br>(61%) | 698±56**<br>(59%) |

## Table 23. Body weight and body weight loss rate of animals in each group during the administration period

| Group | Mean animal body weight±SE (g) or (body weight loss rate) | | | | | | | | |
|---|---|---|---|---|---|---|---|---|---|
| | D12 | D15 | D19 | D22 | D26 | D29 | D33 | D36 | D40 |
| A | 17.6±0.3 | 17.8±0.6 (-0.78%) | 17.9±0.5 (-1.7%) | 18.1±0.4 (-2.4%) | 18.1±0.3 (-2.6%) | 18.4±0.4 (-4.1%) | 18.4±0.4 (-4.5%) | 17.6±0.4 (0.43%) | 17.8±0.4 (-1.1%) |
| B | 17.6±0.3 | 17.2±0.2 (2.6%) | 16.9±0.3 (3.9%) | 16.3±0.3 (7.4%) | 15.8±0.4 (10%) | 15.3±0.3 (13%) | 14.8±0.4 (16%) | 14.8±0.4 (16%) | 14.9±0.4 (15%) |
| C | 17.9±0.3 | 18.1±0.3 (-1.0%) | 17.9±0.2 (-0.35%) | 18.2±0.2 (-1.8%) | 18.3±0.2 (-2.1%) | 18.3±0.2 (-2.5%) | 18.3±0.3 (-2.3%) | 18.0±0.3 (-0.84%) | 17.8±0.3 (0.70%) |
| D | 17.6±0.2 | 17.0±0.2 (3.3%) | 16.6±0.3 (5.5%) | 16.2±0.3 (7.7%) | 15.8±0.3 (10%) | 15.3±0.4 (13%) | 14.6±0.5 (17%) | 14.9±0.4 (15%) | 15.4±0.4 (12%) |

Note: "*" indicates that there was a significant difference in the tumor volume compared with the solvent control group ($P < 0.05$); "**" indicates that there was a highly significant difference in the tumor volume compared with the solvent control group ($P < 0.01$).

It can be seen from the above tables that, the compound of formula (I) of the present disclosure may have a synergistic anti-tumor effect with cisplatin, that is, the combination administration has faster onset of action, better effect, and no significant reduction in safety.

**Example 11: Antagonistic effect of the compound of formula (1) against the toxicity of the chemotherapy drug cisplatin on the main target organ**

Assay purpose

This assay aims to conclusively study whether R01 can significantly reduce the cisplatin-induced acute kidney damage in mice, and to determine the potential of R01 for protecting the cisplatin-induced kidney damage by comparing with amifostine, the most conclusive drug currently used in the first line clinically for antagonising cisplatin-induced kidney damage.

Test samples and reagents

R01 formulation: same as Example 5.
Amifostine trihydrate, batch number: Z28N11R132596, molecular weight: 268.27; content: 98%, Shanghai Yuanye Biotechnology.
Cisplatin (batch number: 601200804, molecular weight: 300.05, purity≥purity≥0508, conversion factor: 1) was purchased from Jiangsu Hansoh Pharmaceutical Group Co., Ltd.

Assay animals

42 male C57BL/6J mice (age: 6 to 8 weeks, body weight 18-20 g, certificate number: 11400700238365) were purchased from Si Pei Fu (Beijing) Biotechnology Co., Ltd., and raised in an SPF-grade animal room of the Institute of Materia Medica, Chinese Academy of Medical Sciences, at a temperature of 20 to 25 °C, a relative humidity of 40% to 70%, and a cycle of 12 hours of light and 12 hours of dark. The animals had free access to water and food. After 3 days of normal feeding, mice with good physical conditions after the veterinary examination were selected for this assay. Before grouping, a marker was used to mark on the base of the animals' tail. After grouping, each animal was marked with an ear cutout (7 mice in each group).

Assay method

Grouping of assay animals

Assay animals: 42 7-week-old male C57 mice were used for animal testing after an adaptive feeding.
Group 1: blank control group;
Group 2: positive control drug cisplatin (10mg/kg);
Group 3: cisplatin (10 mg/kg) + amifostine (50 mg/kg, intraperitoneally injected half an hour before the cisplatin administration, only administered once);
Group 4: cisplatin (10 mg/kg) + R01 (6 mg, bid, administered three days before cisplatin modeling);
Group 5: cisplatin (10 mg/kg) + R01 (18 mg, bid, administered three days before cisplatin modeling);
Group 6: cisplatin (10 mg/kg) + R01 (36 mg, bid, administered three days before cisplatin modeling).

Drug preparation

Preparation of blank solvent: An appropriate amount of sodium carboxymethyl cellulose was taken, and added to physiological saline to prepare a 0.5% solution of sodium carboxymethyl cellulose, which was sterilized by high pressure.

Preparation of cisplatin (DDP) administration test solution: The cisplatin formulation was a solution, which was used directly and stored at room temperature in dark.

The preparation of the R01 formulation administration test solution was the same as that in Example 5: an appropriate amount of the R01 formulation was weighed according to the three dosages set in the assay, respectively, and an appropriate volume of 0.5% CMC-Na aqueous solution was added. The mixture was vortexed and sonicated until mixed evenly, to give the administration test solutions with concentrations of 0.6, 1.8, and 3.6 $mg \cdot kg^{-1}$, respectively, which were ready for use.

Animal administration and sample collection

Animal testing period: 7 days (excluding adaptive feeding), hereinafter referred to as: D1-D7.
Cisplatin administration method: one-time intraperitoneal injection of cisplatin solution (10 mg/kg)
Euthanasia method: fasting 12 hours before euthanasia, and the euthanasia was performed by taking blood from the eyeballs.

1. The first group was a solvent control group: N=7, all mice were males (the same below);

a) 0.5% solution of sodium carboxymethyl cellulose was gavagely administered from D1 to D6 (twice a day for each animal, with an interval of 8 hours between the morning and the afternoon).
b) Physiological saline was intraperitoneally injected in one-time on D4.
c) On D7, blood was collected from the orbit for euthanasia (300ul of serum was extracted), the left kidney was fixed in formaldehyde, and the right kidney was preserved in liquid nitrogen (WB study may be carried out).

2. The second group was a cisplatin-induced kidney damage model group: N=7;

a) sodium carboxymethyl cellulose solution was gavagely administered from D1 to D6 (twice a day for each animal).
b) 10mg/kg of cisplatin was intraperitoneally injected in one-time on D4.
c) On D7, blood was collected from the orbit for euthanasia (300ul of serum was extracted), the left kidney was fixed in formaldehyde, and the right kidney was preserved in liquid nitrogen.

3. The third group was a cisplatin (10 mg/kg) + amifostine (50 mg/kg, intraperitoneally injected half an hour before the cisplatin administration) group: N=7;

a) 10mg/kg of cisplatin was intraperitoneally injected in one-time on D4 (50 mg/kg of amifostine was intraperitoneally injected half an hour before the cisplatin administration).
b) On D7, blood was collected from the orbit for euthanasia (300ul of serum was extracted), the left kidney was fixed in formaldehyde, and the right kidney was preserved in liquid nitrogen.

4. The fourth to the sixth groups were cisplatin (10 mg/kg) + R01 (6, 18, 36 mg/kg, respectively, bid, administered three days before the cisplatin administration) groups: N=7;

a) R01 suspension was gavagely administered from D1 to D6 (twice a day for each animal, with an interval of 8 hours between the morning and the afternoon).
b) 10mg/kg of cisplatin was intraperitoneally injected in one-time on D4.
c) On D7, blood was collected from the orbit for euthanasia (300ul of serum was extracted), the left kidney was fixed in formaldehyde, and the right kidney was preserved in liquid nitrogen.

Testing items

1) The concentrations of urea nitrogen and creatinine in peripheral blood serum were tested.
2) Renal pathology testing: HE, PAS preparation, slide scanning, and report issuance.
3) Each animal was weighed before euthanasia, and the weight was recorded. Two kidneys were weighed as soon as possible after euthanasia, and the weight was recorded. Then, the two kidneys were quickly fixed or cryopreserved, and the kidney coefficient of the mouse was calculated (kidney weight/mouse weight).

Data recording and calculation

The body weight of the animals when being grouped for administration in each group was recorded. The body weight and the kidney weight at the end of the assay were also recorded, and the kidney coefficient was calculated. The creatinine and urea nitrogen values in the blood samples sent for testing were recorded. The kidneys were stained with HE and PAS, and the slides were scanned. A report was issued after the pathological analysis.

Statistical Analysis

The assay data were calculated and processed statistically using Microsoft Office Excel 2007 software. Unless otherwise specified, the data were expressed as mean ± standard error (Mean ± SE), and t-test was used for comparison between two groups.

Assay results

**Body weight of animals in each group before cisplatin modeling (g)**

| Group / Animal No. | Body weight (g) | | | | | |
|---|---|---|---|---|---|---|
| | Normal group | Model group | Amifostine group | R01 6mg/kg | R01 18mg/kg | R01 36mg/kg |
| 1 | 21.1 | 20.9 | 21.8 | 18.4 | 20.2 | 20.9 |
| 2 | 20.3 | 22.1 | 22.4 | 20.6 | 20.9 | 20.3 |
| 3 | 22.1 | 20.5 | 20.2 | 20.2 | 20 | 19.2 |
| 4 | 20.5 | 20.4 | 20.6 | 21 | 20.8 | 21.3 |
| 5 | 20.2 | 21.1 | 20.4 | 20.6 | 20.4 | 20.2 |
| 6 | 21.2 | 21.2 | 20.6 | 20.8 | 20.9 | 21.4 |
| 7 | 20.3 | 21 | 20.5 | 21.2 | 20.6 | 20.4 |
| Before $\bar{X}$ | 20.8 | 21.0 | 20.9 | 20.4 | 20.5 | 20.5 |

**Body weight of animals in each group after the assay (g)**

| Group / Animal No. | Body weight (g) | | | | | |
|---|---|---|---|---|---|---|
| | Normal group | Model group | Amifostine group | R01 6mg/kg | R01 18mg/kg | R01 36mg/kg |
| 1 | 19.2 | 16.8 | 16.3 | 16.9 | 16.3 | 15.4 |
| 2 | 19.8 | 16.3 | 15.8 | 16.7 | 16.5 | 15.7 |
| 3 | 19.9 | 16.8 | 17.2 | 16.2 | 16 | 17.4 |
| 4 | 20.6 | 15.9 | 18 | 14.3 | 16.2 | 16.6 |
| 5 | 19.2 | 17 | 16.7 | 16.3 | 16.7 | 15.7 |
| 6 | 19.2 | 16.5 | 16.8 | 16.6 | 15.6 | 15.9 |
| 7 | 20.1 | 16.9 | 17.1 | 16.4 | 16.2 | 15.9 |
| After $\bar{X}$ | 19.7 | 16.6 | 16.8 | 16.2 | 16.2 | 16.1 |
| Before $\bar{X}$-after $\bar{X}$ | 1.1 | 4.4. | 4.1 | 4.2 | 4.3 | 4.4 |

It can be seen from the changes in animal body weight before and after the assay that, the body weight of the cisplatin modeling group decreased significantly regardless of whether each group was administered with drugs (P < 0.01). There was no significant difference between the amifostine group and the three groups using R01.

**Kidney weight of animals in each group after the assay**

| Group / Animal No. | Kidney weight (g) | | | | | |
|---|---|---|---|---|---|---|
| | Normal group | Model group | Amifostine group | R01 6mg/kg | R01 18mg/kg | R01 36mg/kg |
| 1 | 0.260 | 0.239 | 0.220 | 0.228 | 0.210 | 0.213 |
| 2 | 0.249 | 0.249 | 0.205 | 0.214 | 0.213 | 0.189 |
| 3 | 0.251 | 0.235 | 0.241 | 0.270 | 0.211 | 0.206 |
| 4 | 0.247 | 0.229 | 0.223 | 0.207 | 0.214 | 0.192 |
| 5 | 0.247 | 0.215 | 0.249 | 0.212 | 0.211 | 0.216 |
| 6 | 0.244 | 0.230 | 0.202 | 0.209 | 0.206 | 0.203 |
| 7 | 0.261 | 0.235 | 0.227 | 0.224 | 0.215 | 0.228 |
| $\bar{X}$ | 0.251 | 0.233 | 0.224 | 0.223 | 0.211 | 0.207 |

**Kidney coefficient of animals in each group after the assay**

| Group / Animal No. | Kidney coefficient (kidney weight/body weight) | | | | | |
|---|---|---|---|---|---|---|
| | Normal group | Model group | Amifostine group | R01 6mg/kg | R01 18mg/kg | R01 36mg/kg |
| 1 | 0.014 | 0.015 | 0.014 | 0.014 | 0.013 | 0.013 |
| 2 | 0.013 | 0.016 | 0.013 | 0.013 | 0.014 | 0.013 |
| 3 | 0.014 | 0.015 | 0.015 | 0.018 | 0.014 | 0.014 |
| 4 | 0.014 | 0.014 | 0.014 | 0.013 | 0.014 | 0.012 |
| 5 | 0.013 | 0.013 | 0.015 | 0.013 | 0.013 | 0.014 |
| 6 | 0.013 | 0.014 | 0.013 | 0.015 | 0.013 | 0.014 |
| 7 | 0.013 | 0.014 | 0.014 | 0.014 | 0.013 | 0.014 |
| $\bar{X}$ | 0.013 | 0.015 | 0.014 | 0.014 | 0.013 | 0.013 |

According to the values measured in the assay, the kidney coefficients of the model groups increased significantly **(P < 0.01)**, while there was no significant difference in the drug administration groups. Overall, the kidney coefficient of each drug intervention group was equivalent to that of the blank control group, and there was no significant difference between the amifostine group and the three groups using R01. In particular, the mid- and high-dosage groups of R01 were more similar to the blank control group.

**Serum creatinine (Cre) levels of animals in each group after the assay**

| Group / Animal No. | Unit (mg/dl) | | | | | |
|---|---|---|---|---|---|---|
| | Normal group | Model group | Amifostine group | R01 6mg/kg | R01 18mg/kg | R01 36mg/kg |
| 1 | 16 | 45 | 17 | 18 | 12 | 15 |
| 2 | 15 | 31 | 18 | 5 | 21 | 16 |
| 3 | 12 | 18 | 9 | 20 | 15 | 15 |
| 4 | 15 | 18 | 19 | 23 | 12 | 13 |
| 5 | 19 | 19 | 14 | 26 | 15 | 17 |
| 6 | 15 | 19 | 18 | 17 | 17 | 15 |
| 7 | 17 | 27 | 18 | 20 | 16 | 16 |
| $\bar{X}$ | 15.6 | 25.3 | 16.1 | 18.4 | 15.4 | 15.3 |

According to the values measured in the assay, the serum creatinine value of the cisplatin modeling group was significantly increased **(P < 0.01)**. The serum creatinine value of each drug intervention group was equivalent to that of the blank control group, and there was no significant difference between the amifostine group and the three groups using R01.

**Urea nitrogen (Bun) levels of animals in each group after the assay**

| Group / Animal No. | Unit (mg/dl) | | | | | |
|---|---|---|---|---|---|---|
| | Normal group | Model group | Amifostine group | R01 6mg/kg | R01 18mg/kg | R01 36mg/kg |
| 1 | 9.67 | 48.45 | 7.62 | 13.85 | 10.38 | 9.98 |
| 2 | 13.22 | 21.65 | 8.78 | 4.91 | 22 | 14 |
| 3 | 9.64 | 11.8 | 6.47 | 14.1 | 16.55 | 17.8 |
| 4 | 10.62 | 11.04 | 10.92 | 28.3 | 13.41 | 12.39 |
| 5 | 13.6 | 10.89 | 8.76 | 29.25 | 15.83 | 12.73 |
| 6 | 11.36 | 12.39 | 6.62 | 10.62 | 19.59 | 14.32 |
| 7 | 10.6 | 15.32 | 7.23 | 11.72 | 14.73 | 14.34 |
| $\bar{X}$ | 11.2 | 18.8 | 8.1 | 16.1 | 16.1 | 13.7 |

According to the values measured in the assay, the serum urea nitrogen value of the cisplatin modeling group increased significantly **(P < 0.05)**. The urea nitrogen value of the amifostine group was significantly lower than that of the model group (P < 0.01), and the urea nitrogen value of the R01 high-dosage group was also significantly lower than that of the model group (P<0.05). Although the urea nitrogen concentrations of the R01 low-dosage and medium-dosage groups were lower than that of the model group, no significant difference was found **(P > 0.05)**.

**Pathological damage of animal kidneys**

**Pathological scoring criteria**

| | Grading | Range of lesion involvement |
|---|---|---|
| Tubulointerstitial | 0 | No lesions |
| | 1 | Tubulointerstitial area involved was <10% |
| | 2 | Tubulointerstitial area involved was 10%-25% |
| | 3 | Tubulointerstitial area involved was 25%-46% |
| | 4 | Tubulointerstitial area involved was 46%-75% |
| | 5 | Tubulointerstitial area involved was >75% |

**Pathological scoring results**

| Group \ Animal No. | 1 | 2 | 3 | 4 | 5 | 6 | 7 |
|---|---|---|---|---|---|---|---|
| Blank control group | 0 | 0 | 1 | 0 | 0 | 0 | 0 |
| Positive control group DDP | 2 | 3 | 2 | 4 | 2 | 4 | 2 |
| DDP+amifostine | 2 | 1 | 1 | 1 | 1 | 2 | 1 |
| DDP+R01 (6mg) | 2 | 2 | 1 | 2 | 1 | 3 | 3 |
| DDP+R01 (18mg) | 1 | 1 | 1 | 2 | 1 | 2 | 2 |
| DDP+R01 (36mg) | 1 | 1 | 1 | 1 | 2 | 1 | 1 |

Pathological description and typical pictures

Blank control group: There was basically no damage to the glomeruli and renal tubules of the 7 animals in the blank control group which were raised normally, and the tubular lumen was tight. Only some inflammatory cell infiltration appeared around the glomeruli of some mice, and a typical picture is as shown in Figure 9.

Cisplatin induced kidney damage model group (10mg/kg): This group was the model group, with severe renal function damage in the animals. Biochemical assays showed a significant increase in blood urea nitrogen and creatinine concentrations. From the perspective of pathological characteristics, the pathological damage to the glomeruli was not severe, with only a few animals exhibiting glomerular shrinkage. The main damage was in the renal tubular area, with significant necrosis of renal tubular epithelial cells and the appearance of protein casts. The damage mainly occured in the area of the afferent arterioles. Overall, the renal tubular cells exhibited rough edges and blurred edges, and severe pathological damage were found in some animals. A large number of protein casts were visible throughout the field of view, and a typical picture is as shown in Figure 10.

Cisplatin + amifostine group: The degree of kidney damage in this group was significantly reduced. Only some mice suffered minor damage, while other animals basically had no obvious pathological damage. A typical picture is as shown in Figure 11.

Cisplatin + R01 6mg group: All the animals in this group suffered a certain degree of kidney damage, wherein 2 animals suffered serious kidney damage, and the others were mildly damaged. The damage was mainly in the renal tubule area, and a typical picture is as shown in Figure 12.

Cisplatin + R01 18mg group: There was basically no obvious pathological damage to the kidneys of the animals in this group. Only one animal had obvious damage, and the others had minor damage. A typical picture is as shown in Figure 13.

Cisplatin + R01 36mg group: Only one mouse in this group showed slight pathological damage to the kidney. The pathological structure of all the other animals was intact and the cell structure was clear. A typical picture is as shown in Figure 14.

A total of 6 groups of mice were evaluated for pathological damage in their kidneys. The blank control group had no pathological damage visible to naked eyes and the tissue morphology was complete. The cisplatin kidney damage model group had significant damage visible to naked eyes, with obvious individual differences. There were a certain degree of differences in the degree of damage, and the main pathological damage existed in the renal tubule area. The pathological damage in the cisplatin + amifostine group was significantly reduced, which was significantly different from the positive control cisplatin group ($P<0.01$). The degree of pathological damage was significantly reduced in the cisplatin+R01 (6mg) group and the cisplatin + R01 (18mg) group ($P<0.05$). The cisplatin+R01 (36 mg) group had basically complete kidney tissue morphology, slight damage ($P<0.01$), and the best effect, which was basically similar to the amifostine group.

**Industrial applicability**

The 2,3-dimethoxy-5-methyl-1,4-benzoquinone alkyl alcohol derivative of the present disclosure has excellent anti-tumor effect and excellent safety.

The above content is a further detailed description of the present disclosure in connection with specific alternative embodiments, and it cannot be concluded that the specific embodiments of the present disclosure are limited to these descriptions. For those of ordinary skill in the technical field to which the present disclosure belongs, without departing from the concept of the present disclosure, some simple deductions or substitutions may be made, which should be

regarded as falling within the protection scope of the present disclosure.

## Claims

1. A compound of formula (I), or a solvate, a hydrate, a polymorph, a prodrug or an isotopic variant thereof, or a mixture thereof:

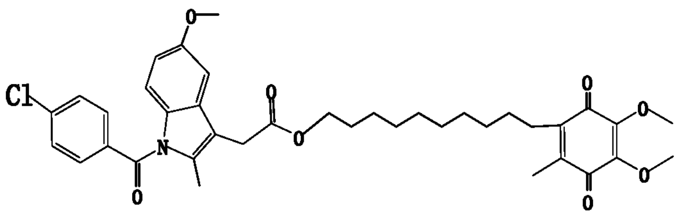

(I).

2. A pharmaceutical composition, comprising the compound of claim 1, or a solvate, a hydrate, a polymorph, a prodrug or an isotopic variant thereof, and pharmaceutically acceptable excipient(s).

3. Use of the compound of claim 1, or a solvate, a hydrate, a polymorph, a prodrug or an isotopic variant thereof, or the pharmaceutical composition of claim 2, in the manufacture of a medicament for the treatment and/or prevention of a cancer.

4. A method of treating and/or preventing a cancer in a subject, comprising administering to the subject the compound of claim 1, or a solvate, a hydrate, a polymorph, a prodrug or an isotopic variant thereof, or the pharmaceutical composition of claim 2.

5. The compound of claim 1, or a solvate, a hydrate, a polymorph, a prodrug or an isotopic variant thereof, or the pharmaceutical composition of claim 2, for use in the treatment and/or prevention of a cancer.

6. The use of claim 3, or the method of claim 4, or the compound or composition for use of claim 5, wherein the cancer is selected from lung cancer, gastric cancer, esophageal cancer, and colorectal cancer.

7. A combination, comprising the compound of formula (I), or a solvate, a hydrate, a polymorph, a prodrug or an isotopic variant thereof, and a platinum-based drug.

8. A pharmaceutical composition, comprising the combination of claim 7 and a pharmaceutically acceptable carrier, diluent or excipient.

9. Use of the combination of claim 7, or the pharmaceutical composition of claim 8, in the manufacture of a medicament for the treatment and/or prevention of a cancer in a subject.

10. A pharmaceutical product, comprising the compound of formula (I), or a solvate, a hydrate, a polymorph, a prodrug or an isotopic variant thereof, and a platinum-based drug, as a combination formulation for simultaneous, sequential or separate use in treatment.

11. A method of treating and/or preventing a cancer in a patient, comprising administering to a subject or patient the compound of formula (I), or a solvate, a hydrate, a polymorph, a prodrug or an isotopic variant thereof, and a platinum-based drug simultaneously, sequentially, or separately.

12. Use of the compound of formula (I), or a solvate, a hydrate, a polymorph, a prodrug or an isotopic variant thereof, and a platinum-based drug, in the manufacture of a medicament for the treatment and/or prevention of a cancer in a subject.

13. Use of the compound of formula (I), or a solvate, a hydrate, a polymorph, a prodrug or an isotopic variant thereof, in the manufacture of a medicament for alleviation of acute kidney damage induced by a platinum-based drug in a subject.

**14.** A method of alleviating acute kidney damage induced by a platinum-based drug in a subject, comprising administering the compound of formula (I), or a solvate, a hydrate, a polymorph, a prodrug or an isotopic variant thereof simultaneously, sequentially or separately with the administration of a platinum-based drug.

**15.** The combination of claim 7, the pharmaceutical composition of claim 8, the pharmaceutical product of claim 10, the method of claim 11, the use of claim 12, the use of claim 13, or the method of claim 14, wherein the platinum-based drug is selected from cisplatin, carboplatin, nedaplatin, oxaliplatin, and loplatin.

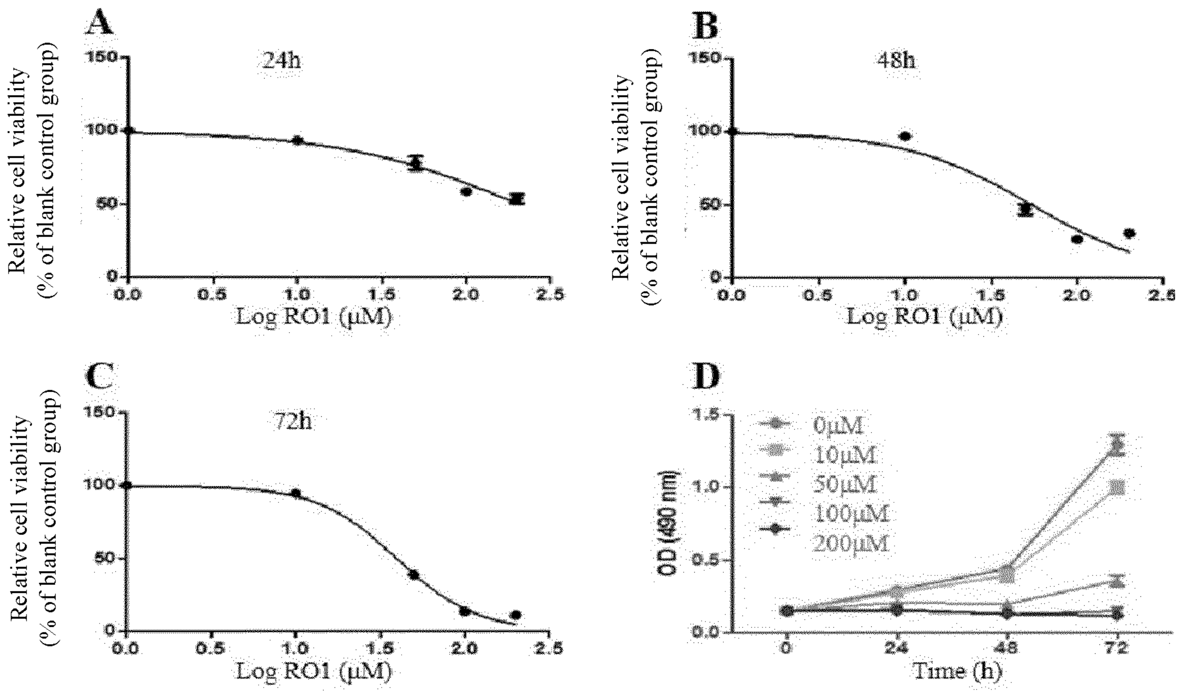
A
24h
Relative cell viability
(% of blank control group)
Log RO1 (μM)
B
48h
C
72h
D
0μM
10μM
50μM
100μM
200μM
OD (490 nm)
Time (h)

FIG. 1

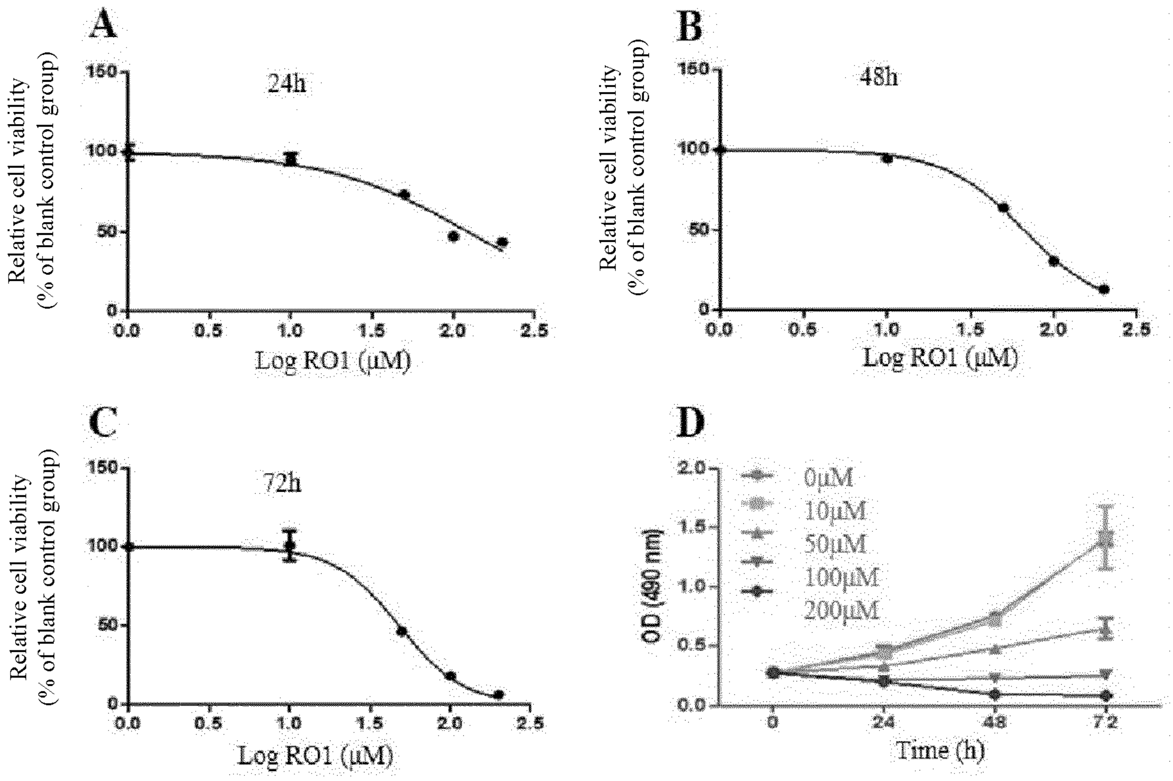
A
24h
Relative cell viability
(% of blank control group)
Log RO1 (μM)
B
48h
C
72h
D
0μM
10μM
50μM
100μM
200μM
OD (490 nm)
Time (h)

FIG. 2

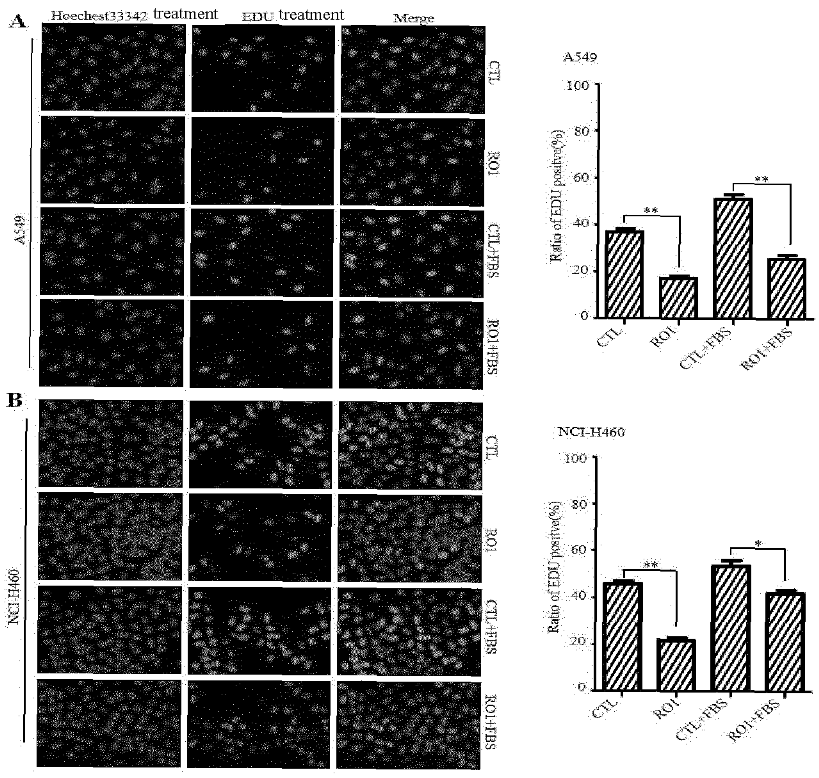
A
Hoechest33342 treatment
EDU treatment
Merge
A549
CTL
R01
CTL+FBS
R01+FBS
B
NCI-H460
A549
Ratio of EDU positive(%)
100
80
60
40
20
0
**
NCI-H460
*

FIG. 3

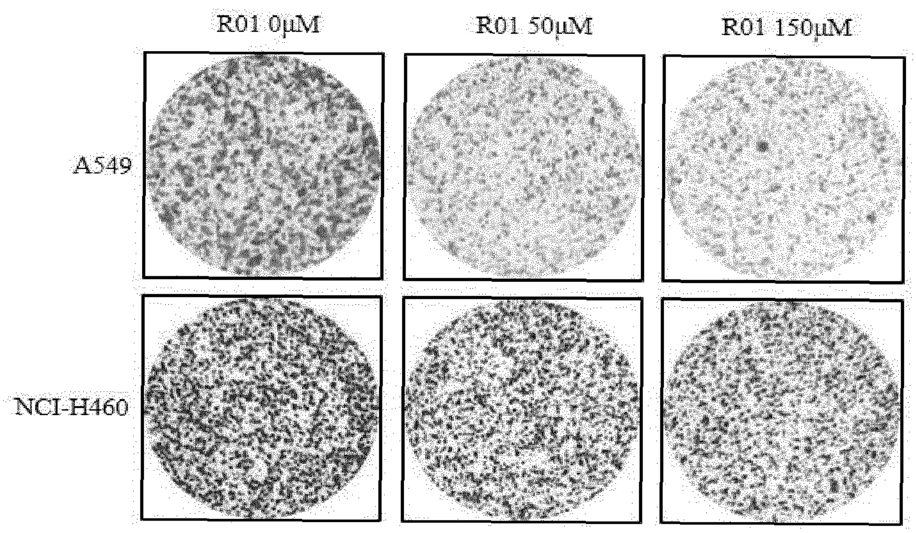
R01 0μM
R01 50μM
R01 150μM
A549
NCI-H460

FIG. 4

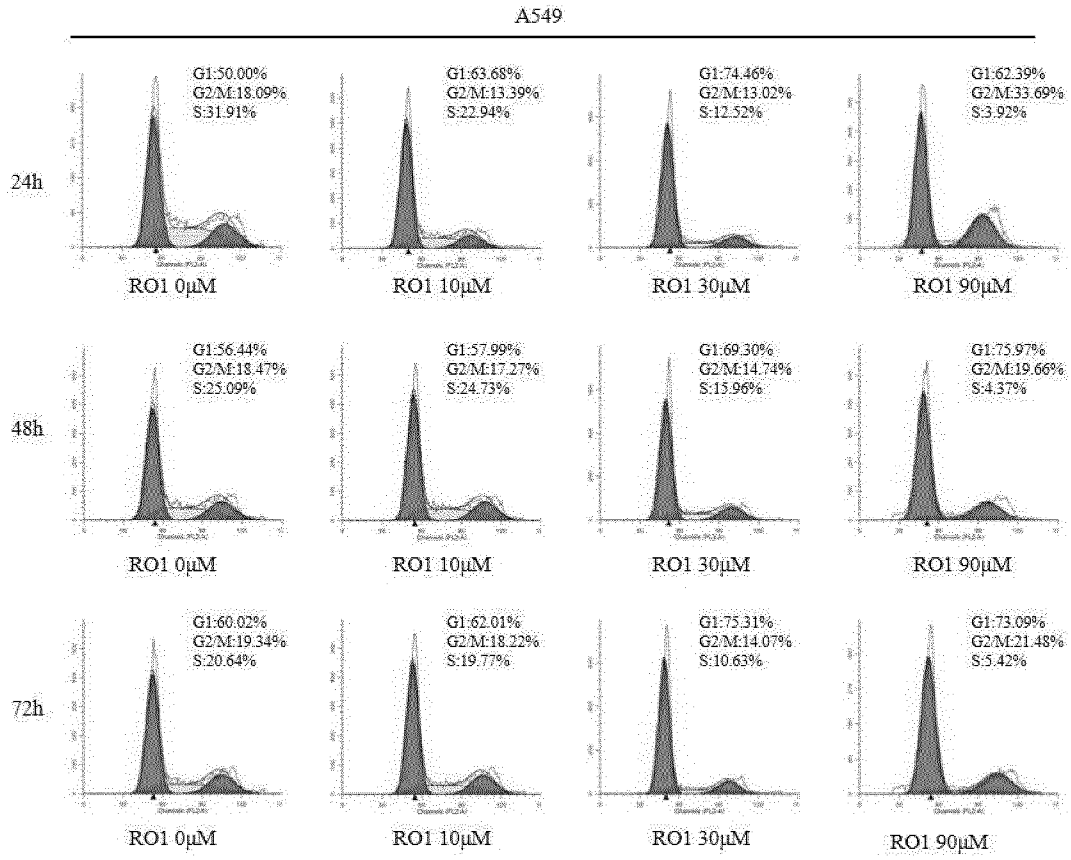
A549
24h
G1:50.00%
G2/M:18.09%
S:31.91%
RO1 0μM
G1:63.68%
G2/M:13.39%
S:22.94%
RO1 10μM
G1:74.46%
G2/M:13.02%
S:12.52%
RO1 30μM
G1:62.39%
G2/M:33.69%
S:3.92%
RO1 90μM
48h
G1:56.44%
G2/M:18.47%
S:25.09%
RO1 0μM
G1:57.99%
G2/M:17.27%
S:24.73%
RO1 10μM
G1:69.30%
G2/M:14.74%
S:15.96%
RO1 30μM
G1:75.97%
G2/M:19.66%
S:4.37%
RO1 90μM
72h
G1:60.02%
G2/M:19.34%
S:20.64%
RO1 0μM
G1:62.01%
G2/M:18.22%
S:19.77%
RO1 10μM
G1:75.31%
G2/M:14.07%
S:10.63%
RO1 30μM
G1:73.09%
G2/M:21.48%
S:5.42%
RO1 90μM

FIG. 5

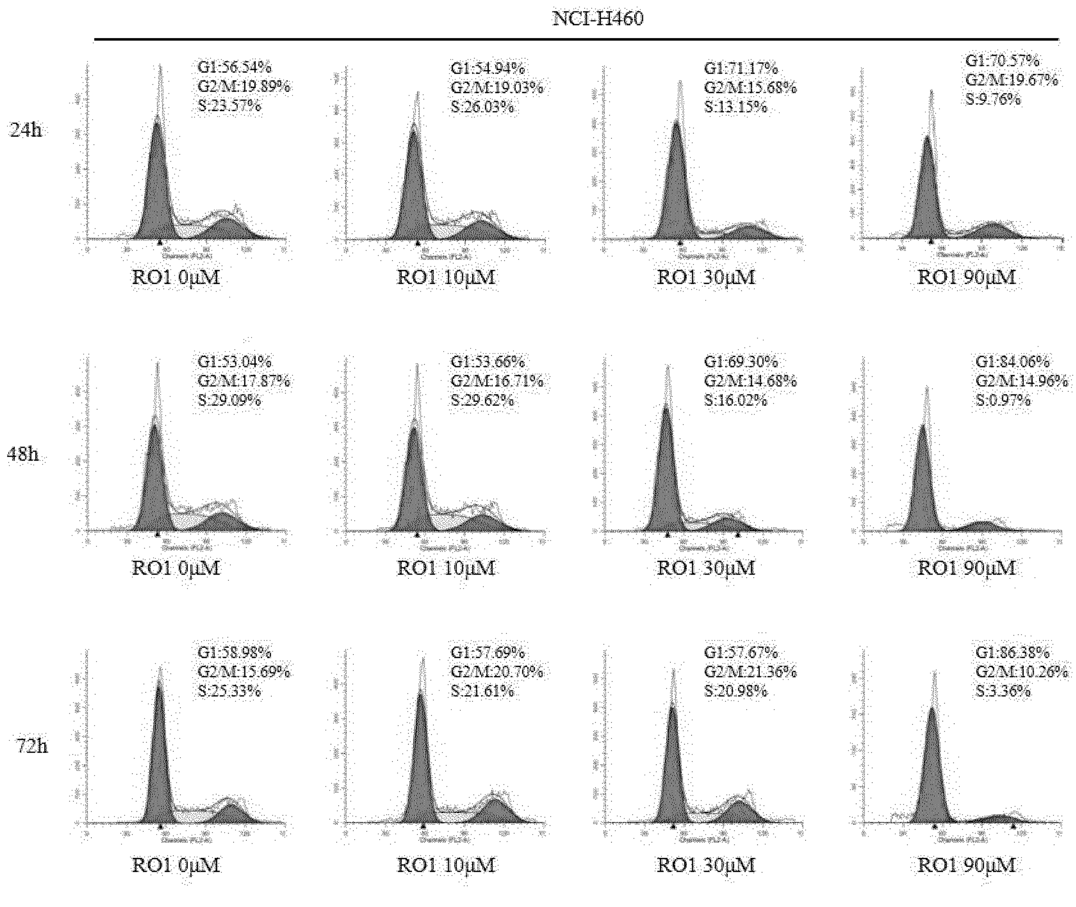
NCI-H460
24h
G1:56.54%
G2/M:19.89%
S:23.57%
RO1 0μM
G1:54.94%
G2/M:19.03%
S:26.03%
RO1 10μM
G1:71.17%
G2/M:15.68%
S:13.15%
RO1 30μM
G1:70.57%
G2/M:19.67%
S:9.76%
RO1 90μM
48h
G1:53.04%
G2/M:17.87%
S:29.09%
RO1 0μM
G1:53.66%
G2/M:16.71%
S:29.62%
RO1 10μM
G1:69.30%
G2/M:14.68%
S:16.02%
RO1 30μM
G1:84.06%
G2/M:14.96%
S:0.97%
RO1 90μM
72h
G1:58.98%
G2/M:15.69%
S:25.33%
RO1 0μM
G1:57.69%
G2/M:20.70%
S:21.61%
RO1 10μM
G1:57.67%
G2/M:21.36%
S:20.98%
RO1 30μM
G1:86.38%
G2/M:10.26%
S:3.36%
RO1 90μM

FIG. 6

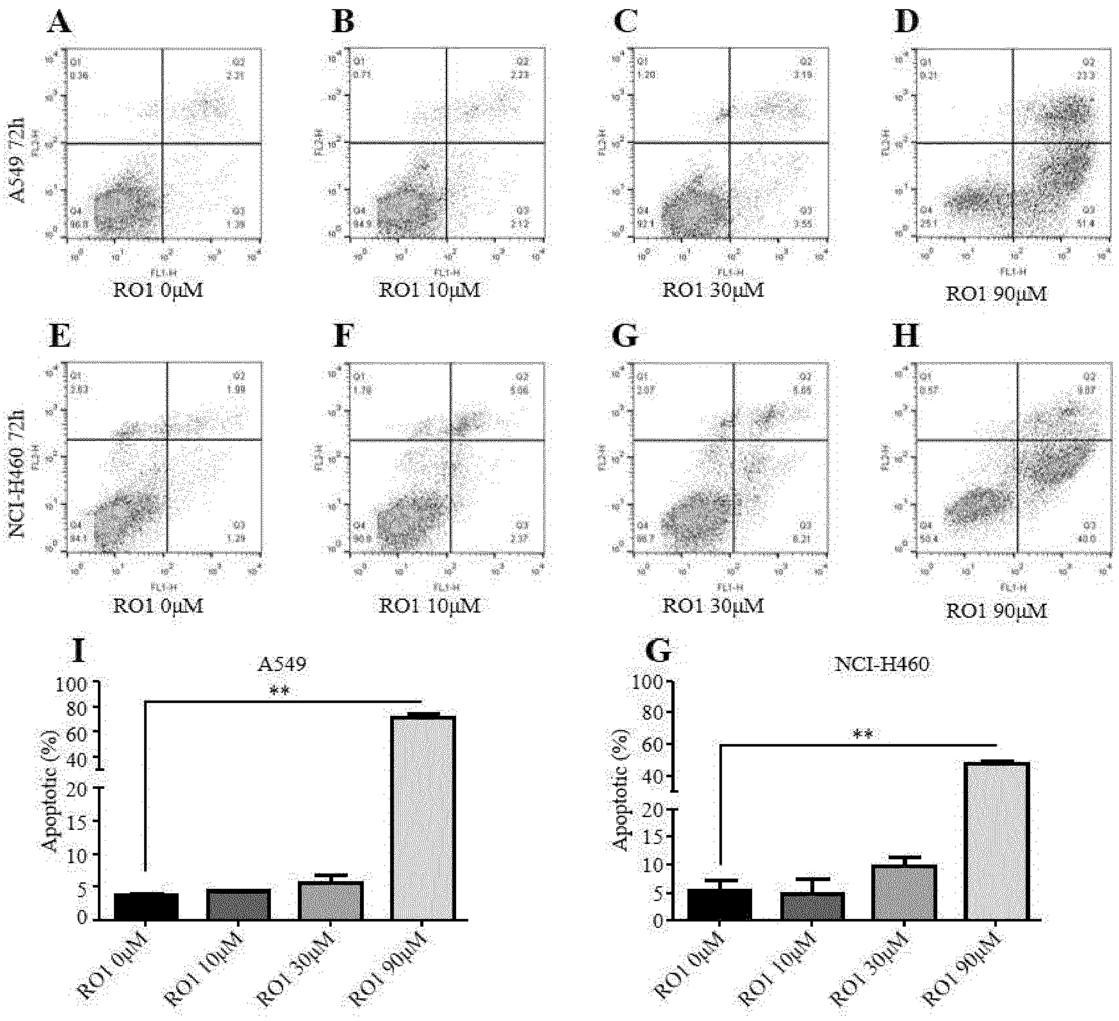
A
B
C
D
A549 72h
RO1 0μM
RO1 10μM
RO1 30μM
RO1 90μM
E
F
G
H
NCI-H460 72h
RO1 0μM
RO1 10μM
RO1 30μM
RO1 90μM
I
A549
**
Apoptotic (%)
RO1 0μM
RO1 10μM
RO1 30μM
RO1 90μM
G
NCI-H460
**
Apoptotic (%)
RO1 0μM
RO1 10μM
RO1 30μM
RO1 90μM

FIG. 7

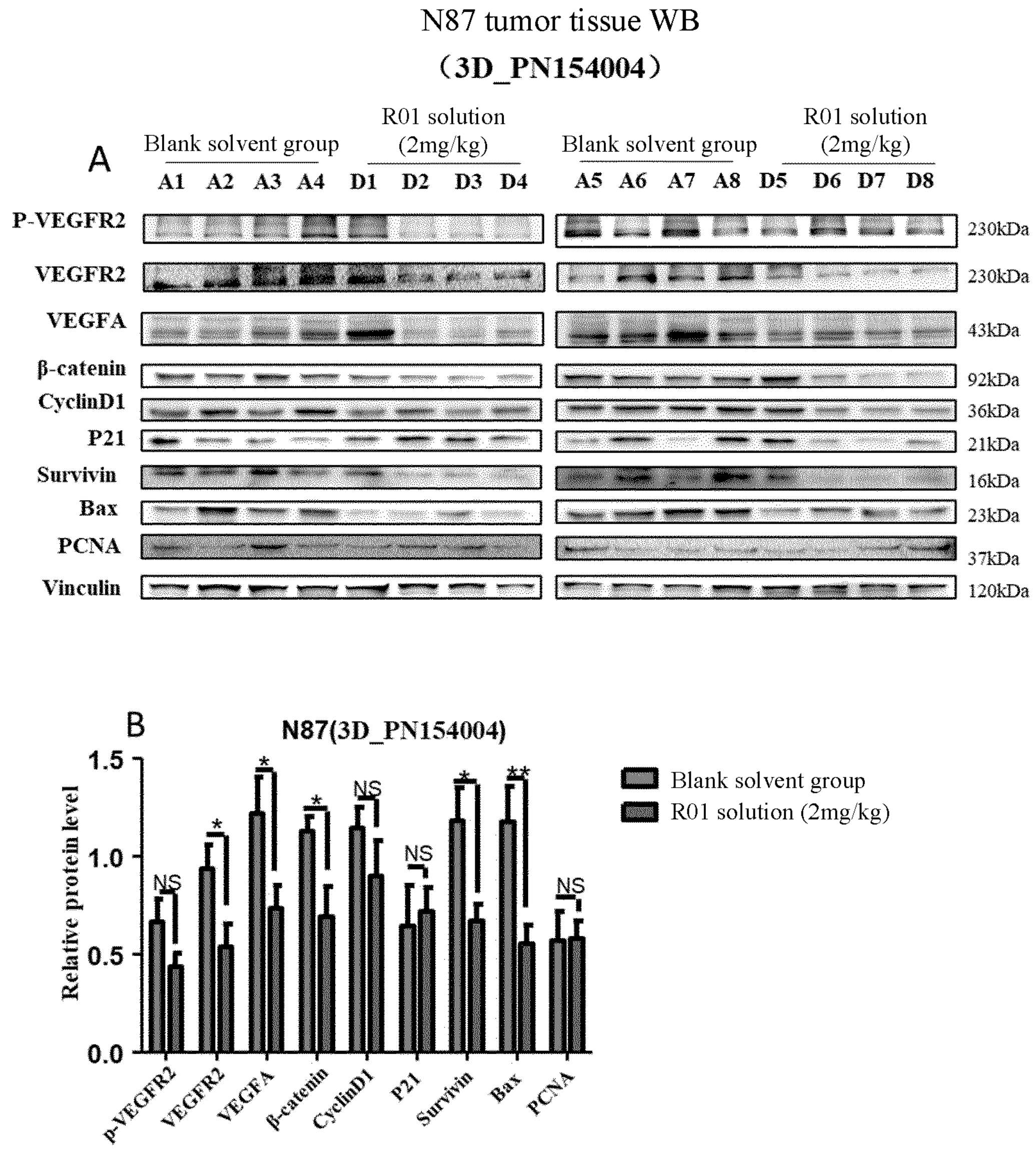
N87 tumor tissue WB
(3D_PN154004)
A
Blank solvent group
R01 solution (2mg/kg)
A1 A2 A3 A4 D1 D2 D3 D4
A5 A6 A7 A8 D5 D6 D7 D8
P-VEGFR2
VEGFR2
VEGFA
β-catenin
CyclinD1
P21
Survivin
Bax
PCNA
Vinculin
230kDa
230kDa
43kDa
92kDa
36kDa
21kDa
16kDa
23kDa
37kDa
120kDa
B
N87(3D_PN154004)
Relative protein level
1.5
1.0
0.5
0.0
NS
*
*
*
NS
NS
*
**
NS
p-VEGFR2
VEGFR2
VEGFA
β-catenin
CyclinD1
P21
Survivin
Bax
PCNA
Blank solvent group
R01 solution (2mg/kg)

FIG. 8

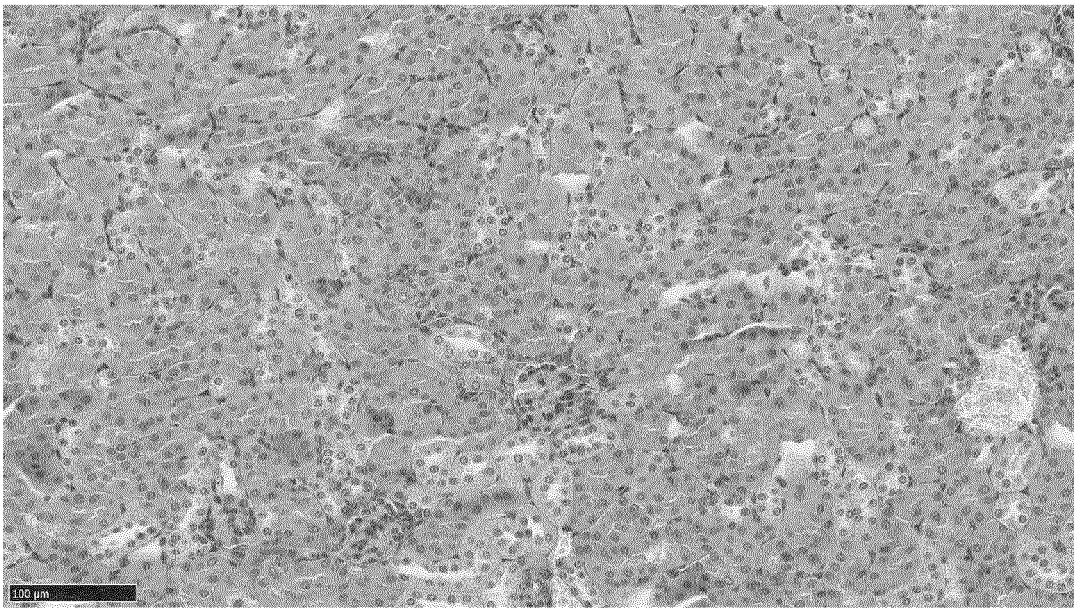


FIG. 9

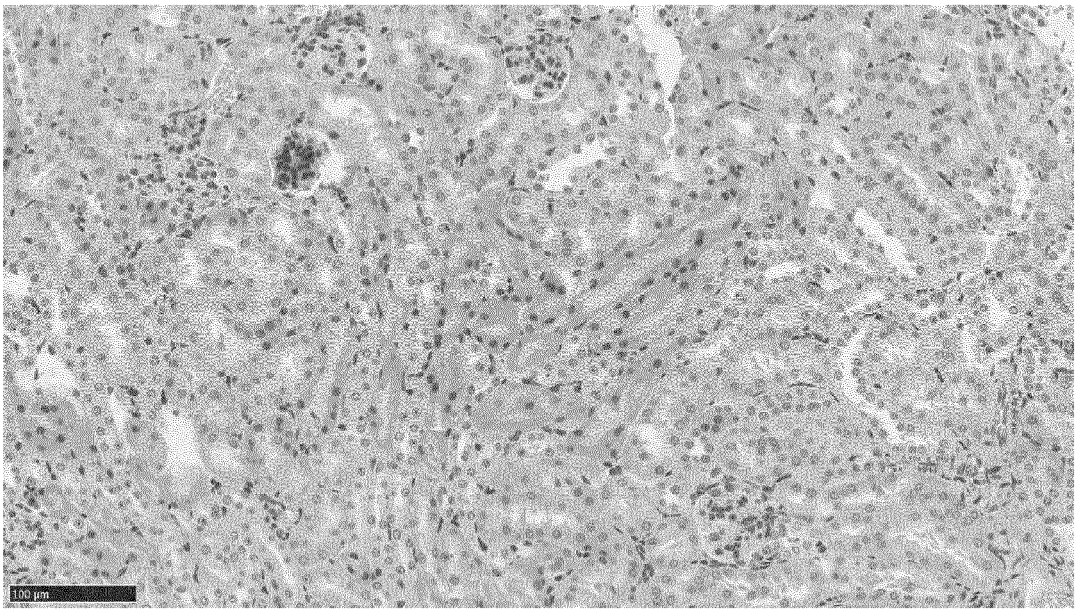


FIG. 10

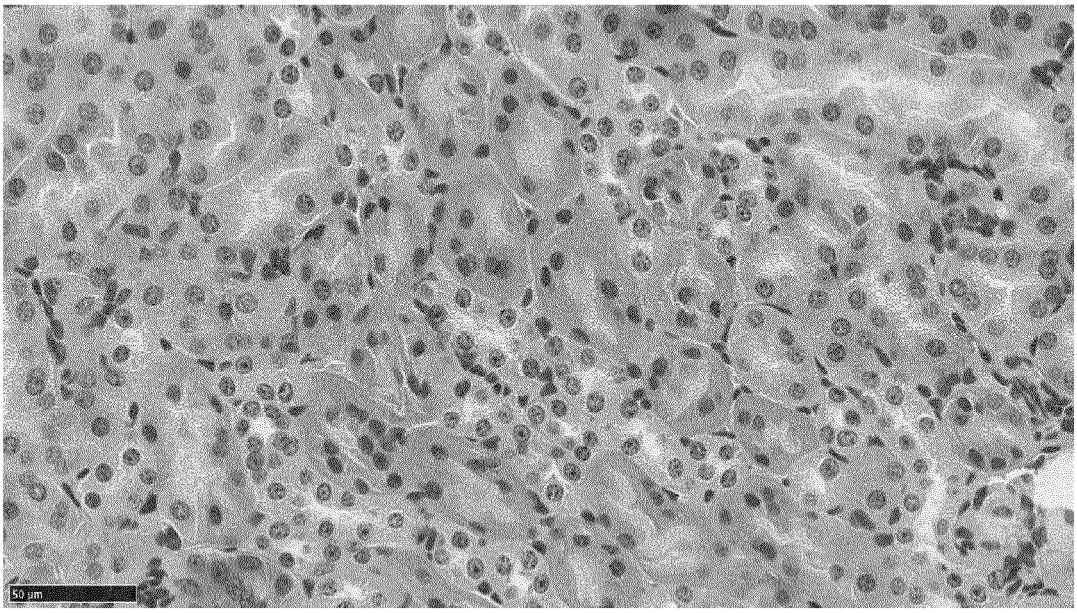


FIG. 11

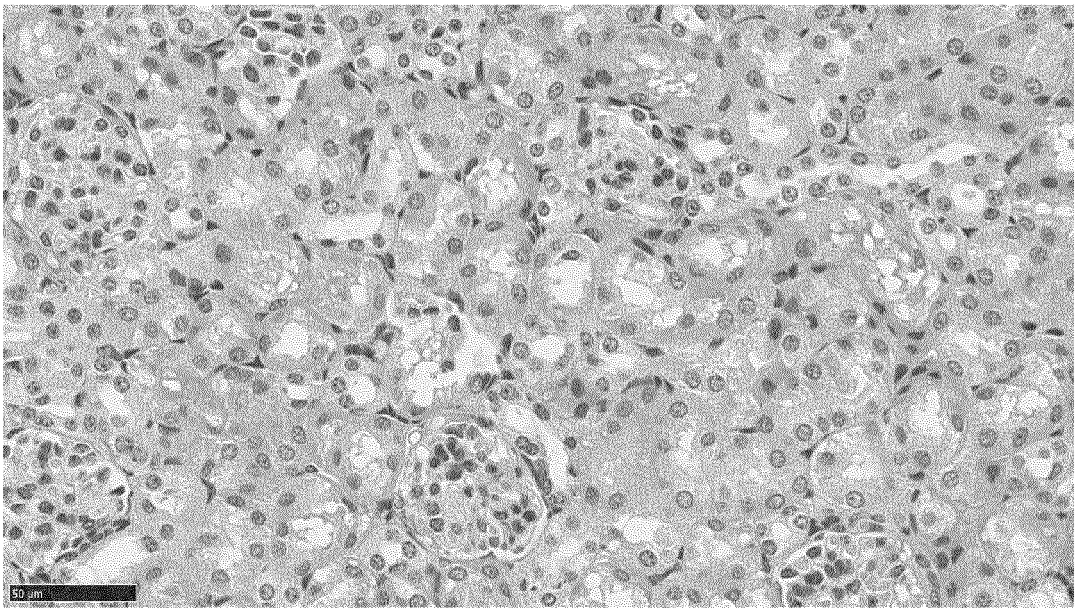


FIG. 12

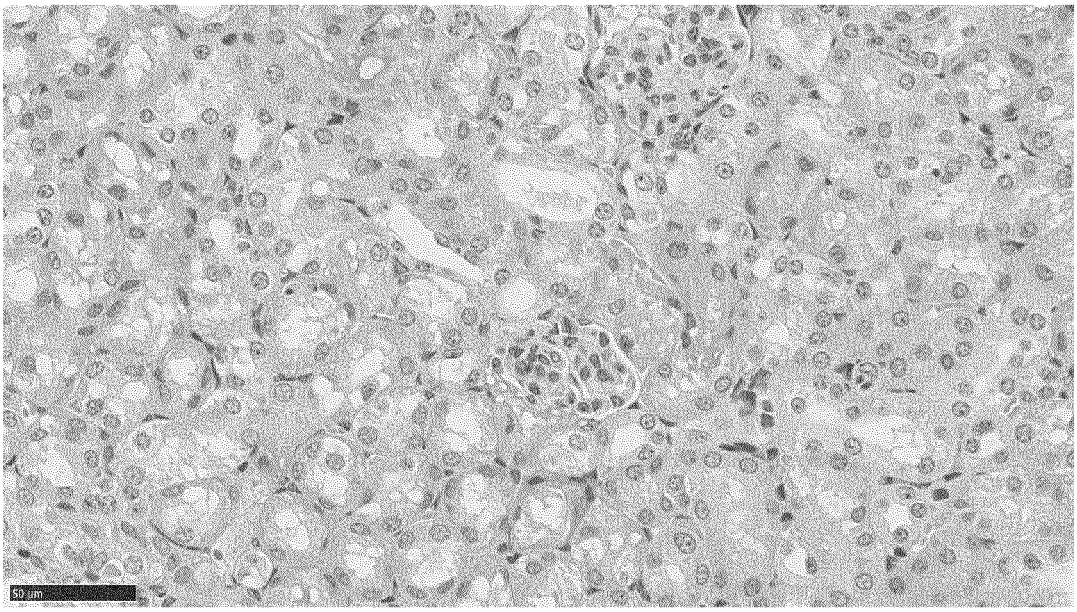


FIG. 13

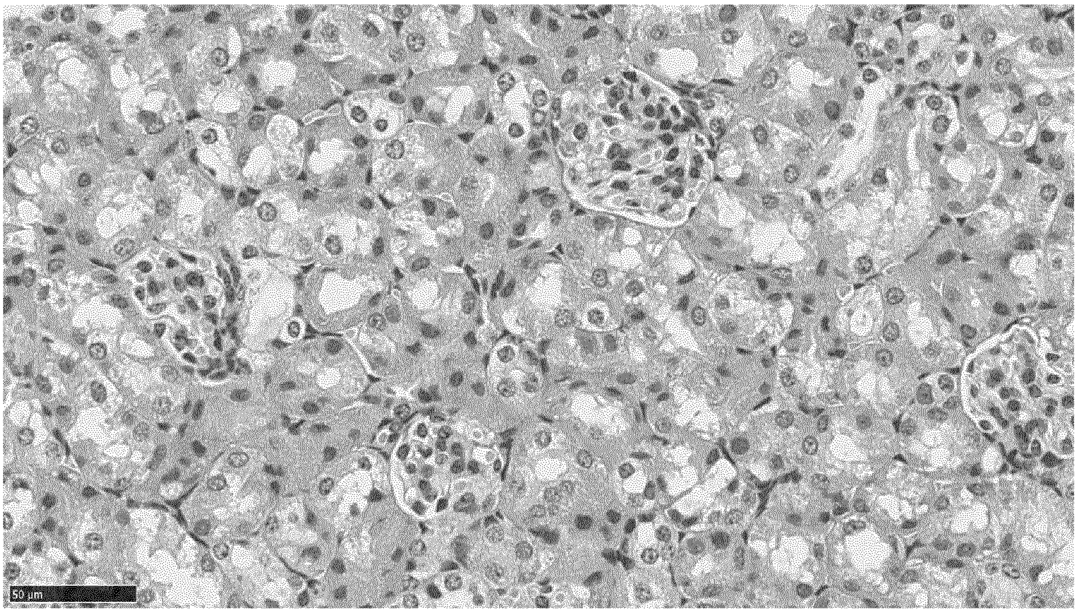


FIG. 14

# INTERNATIONAL SEARCH REPORT

International application No. **PCT/CN2022/125293**

**A. CLASSIFICATION OF SUBJECT MATTER**

C07D 209/26(2006.01)i; C07C 317/44(2006.01)i; A61K 31/405(2006.01)i; A61K 31/216(2006.01)i; A61P 35/00(2006.01)i; A61P 35/02(2006.01)i; A61P 29/00(2006.01)i

According to International Patent Classification (IPC) or to both national classification and IPC

**B. FIELDS SEARCHED**

Minimum documentation searched (classification system followed by classification symbols)

C07D; C07C; A61K; A61P

Documentation searched other than minimum documentation to the extent that such documents are included in the fields searched

Electronic data base consulted during the international search (name of data base and, where practicable, search terms used)

CNKI, CNPAT, WPI, EPODOC, STN: 安健熹医药, 苯醌烷基, 甲基, 醇, 吲哚, 茚, 抑制剂, 癌, 肿瘤, 耐药, sensitive, indole, indene, inhibitor, tumor, cancer, structural formula search, 1770789-09-7/RN

**C. DOCUMENTS CONSIDERED TO BE RELEVANT**

| Category* | Citation of document, with indication, where appropriate, of the relevant passages | Relevant to claim No. |
|---|---|---|
| X | WO 2016119643 A1 (RAN RUIQIONG) 04 August 2016 (2016-08-04)<br>abstract, description, page 2, paragraphs 3-4, embodiment 1, and claims 1-11 | 1-15 |
| A | WO 2006099416 A1 (NITROMED, INC.) 21 September 2006 (2006-09-21)<br>entire document | 1-15 |
| A | US 3654349 A (MERCK & CO., INC. et al.) 04 April 1972 (1972-04-04)<br>entire document | 1-15 |
| A | CN 1237162 A (AMERICAN HOME PRODUCTS CORPORATION) 01 December 1999 (1999-12-01)<br>entire document | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 December 2022** | **16 December 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/125293**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **4,6,11,14-15**
because they relate to subject matter not required to be searched by this Authority, namely:

[1] Claims 4, 6, 11, and 14-15 relate to a disease treatment method. The present search report was made on the basis of an application of a compound in the preparation of drugs.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/125293**

| Patent document cited in search report | Publication date (day/month/year) | Patent family member(s) | Publication date (day/month/year) |
|---|---|---|---|
| WO 2016119643 A1 | 04 August 2016 | KR 20170129688 A | 27 November 2017 |
| | | JP 2018505184 A | 22 February 2018 |
| | | US 2018022700 A1 | 25 January 2018 |
| | | EP 3978473 A1 | 06 April 2022 |
| | | EP 3252039 A1 | 06 December 2017 |
| | | CN 104592091 A | 06 May 2015 |
| | | AU 2016212552 A1 | 17 August 2017 |
| WO 2006099416 A1 | 21 September 2006 | None | |
| US 3654349 A | 04 April 1972 | None | |
| CN 1237162 A | 01 December 1999 | WO 9821195 A1 | 22 May 1998 |
| | | JP 2001504118 A | 27 March 2001 |
| | | KR 20000053227 A | 25 August 2000 |
| | | EP 0948493 A1 | 13 October 1999 |
| | | CA 2269035 A1 | 22 May 1998 |
| | | BR 9713346 A | 09 May 2000 |
| | | HU 9904696 A2 | 28 May 2000 |
| | | AU 5105598 A | 03 June 1998 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Prodrugs as Novel Delivery Systems. **T. HIGUCHI ; V. STELLA.** A.C.S. Symposium Series. American Pharmaceutical Association and Pergamon Press, 1987, vol. 14
- **D. FLEISHER ; S. RAMON ; H. BARBRA.** Improved oral drug delivery: solubility limitations overcome by the use of prodrugs. *Advanced Drug Delivery Reviews,* 1996, vol. 19 (2), 115-130
- Remington's Pharmaceutical Sciences. Mack Publishing Company, 1985
- *Molecular Cancer Research,* June 2020, vol. 18 (6), 926-937